# EUROPEAN PATENT APPLICATION

(11) **EP 1 249 456 A1**
(43) Date of publication of application: **16.10.2002**
(21) Application number: 01900751.7
(22) Date of filing: 15.01.2001
(51) Int. Cl.: C07K 7/08, C07K 16/42, C12P 21/08, C12N 15/13, C12N 5/10, C12N 1/21, A61K 39/395, A61P 37/08

(54) **NOVEL PEPTIDE AND SCREENING METHOD BY USING THE SAME**

(30) Priority: 14.01.2000 JP 2000007061
(71) Applicant: DAIICHI PHARMACEUTICAL CO., LTD., Chuo-ku, Tokyo 103-8234 (JP)
(72) Inventor: WASHIDA, Naohiro, Shimotsuga-gun, Tochigi 329-0518 (JP); TAKAHASHI, Ken, namikawachi-machi, Kawachi-gun, T ochigi (JP); SATAKE, Toshiko, Kawagoe-shi, Saitama 350-0067 (JP); FUJISE, Nobuaki, Shimotuga-gun, Tochigi 329-0511 (JP); TANAKA, Hideki, Omiya-shi, Saitama 330-0033 (JP); KURIYAMA, Masayuki, Omiya-shi, Saitama 330-0021 (JP)
(74) Representative: Wakerley, Helen Rachael
(86) International application number: JP0100181
(87) International publication number: WO01051507

(57) **Abstract**

There is provided a novel peptide, a method of screening using the peptide, and an antibody obtained by the method of screening.

The novel peptide comprises the 18 amino acids described in the Sequence ID No. 1 of the Sequence Table, the method of screening a human-type antihuman IgE monoclonal antibody uses the peptide, and the human-type antihuman IgE monoclonal antibody is obtained by the method of screening.

The antibodies obtained by the present invention are useful as a pharmaceutical for preventing and/or treating allergic diseases.

SEQUENCE LISTING

<110> SNOW BRAND MILK PRODUCTS CO., LTD.

<120> NOVEL PEPTIDE AND SCREENING METHOD USING THE SAME.

<130> SNOW-134

<150> JP7061-2000
<151> 2000-1-14

<160> 23

<170> PatentIn Ver. 2.0

<210> 1
<211> 18
<212> PRT
<213> Artificial Sequence

<220>
<223> Description of Artificial Sequence:Synthesized peptide

<400> 1
Phe Glu Asp Ser Thr Lys Lys Cys Ala Asp Ser Asn Pro Arg Gly Val
 1               5                  10                  15
Ser Ala

<210> 2
<211> 1422
<212> DNA
<213> Artificial Sequence

<220>
<223> Description of Artificial Sequence:Synthesized DNA

<400> 2
atggactgga cctggaggtt cctctttgtg gtggcagcag ctacaggtgt ccagtcgcag 60
gagcagctgg tgcagtctgg ggctgaggtg aagcagactg ggtcctcagt gagagtctcc 120
tgcaaagctt ctggaggcac cttcagcaga tacgccatca actgggtgcg acaggtccct 180
ggacaagggc ttgagtggat ggcagggatc atccctatct ttggtccacc aaagtatgca 240
cagaaattcc agggcagagt ctccctgacc gcggacagat ccacgaacac agcctacatg 300
gagatggccc gcctgaggtc tgacgacacg gccgtgtatt actgtgcgag acgggtggct 360
aggcggttgc gtgctggggc tagtgggttt gactactggg gccagggaac accggtcgcc 420
gtctcctcgg cctccaccaa gggcccatcg gtcttccccc tggcaccctc ctccaagagc 480
acctctgggg gcacagcggc cctgggctgc ctggtcaagg actacttccc cgaaccggtg 540
acggtgtcgt ggaactcagg cgccctgacc agcggcgtgc acaccttccc ggctgtccta 600
cagtcctcag gactctactc cctcagcagc gtggtgaccg tgccctccag cagcttgggc 660
acccagacct acatctgcaa cgtgaatcac aagcccagca acaccaaggt ggacaagaaa 720
gttgagccca aatcttgtga caaaactcac acatgcccac cgtgcccagc acctgaactc 780
ctggggggac cgtcagtctt cctcttcccc ccaaaaccca aggacaccct catgatctcc 840
cggacccctg aggtcacatg cgtggtggtg gacgtgagcc acgaagaccc tgaggtcaag 900
ttcaactggt acgtggacgg cgtggaggtg cataatgcca agacaaagcc gcgggaggag 960
cagtacaaca gcacgtaccg tgtggtcagc gtcctcaccg tcctgcacca ggactggctg 1020
aatggcaagg agtacaagtg caaggtctcc aacaaagccc tcccagcccc catcgagaaa 1080
accatctcca aagccaaagg gcagccccga gaaccacagg tgtacaccct gcccccatcc 1140
cgggatgagc tgaccaagaa ccaggtcagc ctgacctgcc tggtcaaagg cttctatccc 1200
agcgacatcg ccgtggagtg ggagagcaat gggcagccgg agaacaacta caagaccacg 1260
cctcccgtgc tggactccga cggctccttc ttcctctaca gcaagctcac cgtggacaag 1320
agcaggtggc agcaggggaa cgtcttctca tgctccgtga tgcatgaggc tctgcacaac 1380
cactacacgc agaagagcct ctccctgtct ccgggtaaat ga 1422

<210> 3
<211> 708
<212> DNA
<213> Artificial Sequence

<220>
<223> Description of Artificial Sequence:Synthesized DNA

<400> 3
atgacctgct cccctctcct cctcaccctt ctcattcact gcacagggtc gtgggcccag 60

tctgtgttga cgcagccgcc ctcagtgtct gcggccccag gacagaaggt caccatctcc 120
tgctctggaa ggagctccac cattgcagtt aattctgttt cctggtacca gctgctccca 180
ggatcagccc ccaaactcct catttatgac actactaagc gaccctcagg gattcctgac 240
cgattctctg ggtccaagtc tggcacgtcg gccaccctgg gcatcaccgg actccggact 300
ggggacgagg ccgtttatta ctgcagtgtg gtgcggtata attatgtgcg gctgcggttc 360
ggaggaggca cccacctgac cgtcctcggt cagcccaagg cggcgccctc ggtcactctg 420
ttcccaccct cctctgagga gcttcaagcc aacaaggcca cactggtgtg tctcataagt 480
gacttctacc ctggagccgt gacagtggcc tggaaggcag atagcagccc cgtcaaggcg 540
ggagtggaga ccaccacacc ctccaaacaa agcaacaaca agtacgcggc cagcagctac 600
ctgagcctga cgcctgagca gtggaagtcc cacaaaagct acagctgcca ggtcacgcat 660
gaagggagca ccgtggagaa gacagtggcc cctacagaat gctcatag 708

<210> 4
<211> 1422
<212> DNA
<213> Artificial Sequence

<220>
<223> Description of Artificial Sequence:Synthesized DNA

<400> 4
atggactgga cctggaggtt cctctttgtg gtggcagcag ctacaggtgt ccagtcgcag 60
gagcagctgg tgcagtctgg ggctgaggtg aagcagactg ggtcctcagt gagagtctcc 120
tgcaaagctt ctggaggcac cttcagcaga tacgccatca actgggtgcg acaggtccct 180
ggacaagggc ttgagtggat ggcagggatc atccctatct ttggtccacc aaagtatgca 240
cagaaattcc agggcagagt ctccctgacc gcggacagat ccacgaacac agcctacatg 300
gagatggccc gcctgaggtc tgacgacacg gccgtgtatt actgtgcgag accggggagg 360
tttccgttgt atagggattg gcctgggttt gactactggg gccagggaac accggtcgcc 420
gtctcctcgg cctccaccaa gggcccatcg gtcttccccc tggcaccctc ctccaagagc 480
acctctgggg gcacagcggc cctgggctgc ctggtcaagg actacttccc cgaaccggtg 540
acggtgtcgt ggaactcagg cgccctgacc agcggcgtgc acaccttccc ggctgtccta 600
cagtcctcag gactctactc cctcagcagc gtggtgaccg tgccctccag cagcttgggc 660
acccagacct acatctgcaa cgtgaatcac aagcccagca acaccaaggt ggacaagaaa 720
gttgagccca aatcttgtga caaaactcac acatgcccac cgtgcccagc acctgaactc 780
ctggggggac cgtcagtctt cctcttcccc ccaaaaccca aggacaccct catgatctcc 840
cggacccctg aggtcacatg cgtggtggtg gacgtgagcc acgaagaccc tgaggtcaag 900

ttcaactggt acgtggacgg cgtggaggtg cataatgcca agacaaagcc gcgggaggag 960
cagtacaaca gcacgtaccg tgtggtcagc gtcctcaccg tcctgcacca ggactggctg 1020
aatggcaagg agtacaagtg caaggtctcc aacaaagccc tcccagcccc catcgagaaa 1080
accatctcca aagccaaagg gcagccccga gaaccacagg tgtacaccct gcccccatcc 1140
cgggatgagc tgaccaagaa ccaggtcagc ctgacctgcc tggtcaaagg cttctatccc 1200
agcgacatcg ccgtggagtg ggagagcaat gggcagccgg agaacaacta caagaccacg 1260
cctcccgtgc tggactccga cggctccttc ttcctctaca gcaagctcac cgtggacaag 1320
agcaggtggc agcaggggaa cgtcttctca tgctccgtga tgcatgaggc tctgcacaac 1380
cactacacgc agaagagcct ctccctgtct ccgggtaaat ga 1422

<210> 5
<211> 1422
<212> DNA
<213> Artificial Sequence

<220>
<223> Description of Artificial Sequence:Synthesized DNA

<400> 5
atggactgga cctggaggtt cctctttgtg gtggcagcag ctacaggtgt ccagtcgcag 60
gagcagctgg tgcagtctgg ggctgaggtg aagcagactg ggtcctcagt gagagtctcc 120
tgcaaagctt ctggaggcac cttcagcaga tacgccatca actgggtgcg acaggtccct 180
ggacaagggc ttgagtggat ggcagggatc atccctatct ttggtccacc aaagtatgca 240
cagaaattcc agggcagagt ctccctgacc gcggacagat ccacgaacac agcctacatg 300
gagatggccc gcctgaggtc tgacgacacg gccgtgtatt actgtgcgag accggggagg 360
tttccgttgt atagggattg gcctgggttt gactactggg gccagggaac accggtcgcc 420
gtctcctcgg cctccaccaa gggcccatcg gtcttccccc tggcaccctc ctccaagagc 480
acctctgggg gcacagcggc cctgggctgc ctggtcaagg actacttccc cgaaccggtg 540
acggtgtcgt ggaactcagg cgccctgacc agcggcgtgc acaccttccc ggctgtccta 600
cagtcctcag gactctactc cctcagcagc gtggtgaccg tgccctccag cagcttgggc 660
acccagacct acatctgcaa cgtgaatcac aagcccagca acaccaaggt ggacaagaaa 720
gttgagccca aatcttgtga caaaactcac acatgcccac cgtgcccagc acctgaactc 780
ctggggggac cgtcagtctt cctcttcccc ccaaaaccca aggacaccct catgatctcc 840
cggacccctg aggtcacatg cgtggtggtg gacgtgagcc acgaagaccc tgaggtcaag 900
ttcaactggt acgtggacgg cgtggaggtg cataatgcca agacaaagcc gcgggaggag 960
cagtacaaca gcacgtaccg tgtggtcagc gtcctcaccg tcctgcacca ggactggctg 1020

aatggcaagg agtacaagtg caaggtctcc aacaaagccc tcccagcccc catcgagaaa 1080
accatctcca aagccaaagg gcagccccga gaaccacagg tgtacaccct gcccccatcc 1140
cgggatgagc tgaccaagaa ccaggtcagc ctgacctgcc tggtcaaagg cttctatccc 1200
agcgacatcg ccgtggagtg ggagagcaat gggcagccgg agaacaacta caagaccacg 1260
cctcccgtgc tggactccga cggctccttc ttcctctaca gcaagctcac cgtggacaag 1320
agcaggtggc agcaggggaa cgtcttctca tgctccgtga tgcatgaggc tctgcacaac 1380
cactacacgc agaagagcct ctccctgtct ccgggtaaat ga 1422

<210> 6
<211> 1422
<212> DNA
<213> Artificial Sequence

<220>
<223> Description of Artificial Sequence:Synthesized DNA

<400> 6
atggactgga cctggaggtt cctctttgtg gtggcagcag ctacaggtgt ccagtcgcag 60
gagcagctgg tgcagtctgg ggctgaggtg aagcagactg ggtcctcagt gagagtctcc 120
tgcaaagctt ctggaggcac cttcagcaga tacgccatca actgggtgcg acaggtccct 180
ggacaagggc ttgagtggat ggcagggatc atccctatct ttggtccacc aaagtatgca 240
cagaaattcc agggcagagt ctccctgacc gcggacagat ccacgaacac agcctacatg 300
gagatggccc gcctgaggtc tgacgacacg gccgtgtatt actgtgcgag agctccgact 360
cctttgtatg atacggagac ggggccgtgg ggccagggaa caccggtcgc ctttgactac 420
gtctcctcgg cctccaccaa gggcccatcg gtcttccccc tggcaccctc ctccaagagc 480
acctctgggg gcacagcggc cctgggctgc ctggtcaagg actacttccc cgaaccggtg 540
acggtgtcgt ggaactcagg cgccctgacc agcggcgtgc acaccttccc ggctgtccta 600
cagtcctcag gactctactc cctcagcagc gtggtgaccg tgccctccag cagcttgggc 660
acccagacct acatctgcaa cgtgaatcac aagcccagca acaccaaggt ggacaagaaa 720
gttgagccca aatcttgtga caaaactcac acatgcccac cgtgcccagc acctgaactc 780
ctggggggac cgtcagtctt cctcttcccc ccaaaaccca aggacaccct catgatctcc 840
cggacccctg aggtcacatg cgtggtggtg gacgtgagcc acgaagaccc tgaggtcaag 900
ttcaactggt acgtggacgg cgtggaggtg cataatgcca agacaaagcc gcgggaggag 960
cagtacaaca gcacgtaccg tgtggtcagc gtcctcaccg tcctgcacca ggactggctg 1020
aatggcaagg agtacaagtg caaggtctcc aacaaagccc tcccagcccc catcgagaaa 1080
accatctcca aagccaaagg gcagccccga gaaccacagg tgtacaccct gcccccatcc 1140

cgggatgagc tgaccaagaa ccaggtcagc ctgacctgcc tggtcaaagg cttctatccc 1200
agcgacatcg ccgtggagtg ggagagcaat gggcagccgg agaacaacta caagaccacg 1260
cctcccgtgc tggactccga cggctccttc ttcctctaca gcaagctcac cgtggacaag 1320
agcaggtggc agcaggggaa cgtcttctca tgctccgtga tgcatgaggc tctgcacaac 1380
cactacacgc agaagagcct ctccctgtct ccgggtaaat ga 1422

<210> 7
<211> 708
<212> DNA
<213> Artificial Sequence

<220>
<223> Description of Artificial Sequence:Synthesized DNA

<400> 7
atgacctgct cccctctcct cctcaccctt ctcattcact gcacagggtc gtgggcccag 60
tctgtgttga cgcagccgcc ctcagtgtct gcggccccag gacagaaggt caccatctcc 120
tgctctggaa ggagctccac cattgcagtt aattctgttt cctggtacca gctgctccca 180
ggatcagccc ccaaactcct catttatgac actactaagc gaccctcagg gattcctgac 240
cgattctctg ggtccaagtc tggcacgtcg gccaccctgg gcatcaccgg actccggact 300
ggggacgagg ccgtttatta ctgcgcgctg ttggaggagg gtgcgaaggg gccggagttc 360
ggaggaggca cccacctgac cgtcctcggt cagcccaagg cggcgccctc ggtcactctg 420
ttcccaccct cctctgagga gcttcaagcc aacaaggcca cactggtgtg tctcataagt 480
gacttctacc ctggagccgt gacagtggcc tggaaggcag atagcagccc cgtcaaggcg 540
ggagtggaga ccaccacacc ctccaaacaa agcaacaaca agtacgcggc cagcagctac 600
ctgagcctga cgcctgagca gtggaagtcc cacaaaagct acagctgcca ggtcacgcat 660
gaagggagca ccgtggagaa gacagtggcc cctacagaat gctcatag 708

<210> 8
<211> 1422
<212> DNA
<213> Artificial Sequence

<220>
<223> Description of Artificial Sequence:Synthesized DNA

<400> 8
atggactgga cctggaggtt cctctttgtg gtggcagcag ctacaggtgt ccagtcgcag 60
gagcagctgg tgcagtctgg ggctgaggtg aagcagactg ggtcctcagt gagagtctcc 120
tgcaaagctt ctggaggcac cttcagcaga tacgccatca actgggtgcg acaggtccct 180
ggacaagggc ttgagtggat ggcagggatc atccctatct ttggtccacc aaagtatgca 240
cagaaattcc agggcagagt ctccctgacc gcggacagat ccacgaacac agcctacatg 300
gagatggccc gcctgaggtc tgacgacacg gccgtgtatt actgtgcgag accggtgcct 360
gcgacttatg agtctactgc tggttatttt gactactggg gccagggaac accggtcgcc 420
gtctcctcgg cctccaccaa gggcccatcg gtcttccccc tggcaccctc ctccaagagc 480
acctctgggg gcacagcggc cctgggctgc ctggtcaagg actacttccc cgaaccggtg 540
acggtgtcgt ggaactcagg cgccctgacc agcggcgtgc acaccttccc ggctgtccta 600
cagtcctcag gactctactc cctcagcagc gtggtgaccg tgccctccag cagcttgggc 660
acccagacct acatctgcaa cgtgaatcac aagcccagca acaccaaggt ggacaagaaa 720
gttgagccca aatcttgtga caaaactcac acatgcccac cgtgcccagc acctgaactc 780
ctggggggac cgtcagtctt cctcttcccc ccaaaaccca aggacaccct catgatctcc 840
cggacccctg aggtcacatg cgtggtggtg gacgtgagcc acgaagaccc tgaggtcaag 900
ttcaactggt acgtggacgg cgtggaggtg cataatgcca agacaaagcc gcgggaggag 960
cagtacaaca gcacgtaccg tgtggtcagc gtcctcaccg tcctgcacca ggactggctg 1020
aatggcaagg agtacaagtg caaggtctcc aacaaagccc tcccagcccc catcgagaaa 1080
accatctcca aagccaaagg gcagccccga gaaccacagg tgtacaccct gcccccatcc 1140
cgggatgagc tgaccaagaa ccaggtcagc ctgacctgcc tggtcaaagg cttctatccc 1200
agcgacatcg ccgtggagtg ggagagcaat gggcagccgg agaacaacta caagaccacg 1260
cctcccgtgc tggactccga cggctccttc ttcctctaca gcaagctcac cgtggacaag 1320
agcaggtggc agcaggggaa cgtcttctca tgctccgtga tgcatgaggc tctgcacaac 1380
cactacacgc agaagagcct ctccctgtct ccgggtaaat ga 1422

<210> 9
<211> 708
<212> DNA
<213> Homo sapiens

<220>
<223> Description of Artificial Sequence:Synthesized DNA

<400> 9
atgacctgct cccctctcct cctcaccctt ctcattcact gcacagggtc gtgggcccag 60

tctgtgttga cgcagccgcc ctcagtgtct gcggccccag gacagaaggt caccatctcc 120
tgctctggaa ggagctccac cattgcagtt aattctgttt cctggtacca gctgctccca 180
ggatcagccc ccaaactcct catttatgac actactaagc gaccctcagg gattcctgac 240
cgattctctg ggtccaagtc tggcacgtcg gccaccctgg gcatcaccgg actccggact 300
ggggacgagg ccgtttatta ctgcagtccg gtgcagcggt attctggggg ttctctgttc 360
ggaggaggca cccacctgac cgtcctcggt cagcccaagg cggcgccctc ggtcactctg 420
ttcccaccct cctctgagga gcttcaagcc aacaaggcca cactggtgtg tctcataagt 480
gacttctacc ctggagccgt gacagtggcc tggaaggcag atagcagccc cgtcaaggcg 540
ggagtggaga ccaccacacc ctccaaacaa agcaacaaca agtacgcggc cagcagctac 600
ctgagcctga cgcctgagca gtggaagtcc cacaaaagct acagctgcca ggtcacgcat 660
gaagggagca ccgtggagaa gacagtggcc cctacagaat gctcatag 708

<210> 10
<211> 473
<212> PRT
<213> Homo sapiens

<400> 10
Met Asp Trp Thr Trp Arg Phe Leu Phe Val Val Ala Ala Ala Thr Gly
1               5                   10                  15
Val Gln Ser Gln Glu Gln Leu Val Gln Ser Gly Ala Glu Val Lys Gln
            20                  25                  30
Thr Gly Ser Ser Val Arg Val Ser Cys Lys Ala Ser Gly Gly Thr Phe
        35                  40                  45
Ser Arg Tyr Ala Ile Asn Trp Val Arg Gln Val Pro Gly Gln Gly Leu
    50                  55                  60
Glu Trp Met Ala Gly Ile Ile Pro Ile Phe Gly Pro Pro Lys Tyr Ala
65                  70                  75                  80
Gln Lys Phe Gln Gly Arg Val Ser Leu Thr Ala Asp Arg Ser Thr Asn
                85                  90                  95
Thr Ala Tyr Met Glu Met Ala Arg Leu Arg Ser Asp Asp Thr Ala Val
            100                 105                 110
Tyr Tyr Cys Ala Arg Arg Val Ala Arg Arg Leu Arg Ala Gly Ala Ser
        115                 120                 125
Gly Phe Asp Tyr Trp Gly Gln Gly Thr Pro Val Ala Val Ser Ser Ala
    130                 135                 140

Ser Thr Lys Gly Pro Ser Val Phe Pro Leu Ala Pro Ser Ser Lys Ser
145                 150                 155                 160
Thr Ser Gly Gly Thr Ala Ala Leu Gly Cys Leu Val Lys Asp Tyr Phe
                165                 170                 175
Pro Glu Pro Val Thr Val Ser Trp Asn Ser Gly Ala Leu Thr Ser Gly
            180                 185                 190
Val His Thr Phe Pro Ala Val Leu Gln Ser Ser Gly Leu Tyr Ser Leu
        195                 200                 205
Ser Ser Val Val Thr Val Pro Ser Ser Ser Leu Gly Thr Gln Thr Tyr
    210                 215                 220
Ile Cys Asn Val Asn His Lys Pro Ser Asn Thr Lys Val Asp Lys Lys
225                 230                 235                 240
Val Glu Pro Lys Ser Cys Asp Lys Thr His Thr Cys Pro Pro Cys Pro
                245                 250                 255
Ala Pro Glu Leu Leu Gly Gly Pro Ser Val Phe Leu Phe Pro Pro Lys
            260                 265                 270
Pro Lys Asp Thr Leu Met Ile Ser Arg Thr Pro Glu Val Thr Cys Val
        275                 280                 285
Val Val Asp Val Ser His Glu Asp Pro Glu Val Lys Phe Asn Trp Tyr
    290                 295                 300
Val Asp Gly Val Glu Val His Asn Ala Lys Thr Lys Pro Arg Glu Glu
305                 310                 315                 320
Gln Tyr Asn Ser Thr Tyr Arg Val Val Ser Val Leu Thr Val Leu His
                325                 330                 335
Gln Asp Trp Leu Asn Gly Lys Glu Tyr Lys Cys Lys Val Ser Asn Lys
            340                 345                 350
Ala Leu Pro Ala Pro Ile Glu Lys Thr Ile Ser Lys Ala Lys Gly Gln
        355                 360                 365
Pro Arg Glu Pro Gln Val Tyr Thr Leu Pro Pro Ser Arg Asp Glu Leu
    370                 375                 380
Thr Lys Asn Gln Val Ser Leu Thr Cys Leu Val Lys Gly Phe Tyr Pro
385                 390                 395                 400
Ser Asp Ile Ala Val Glu Trp Glu Ser Asn Gly Gln Pro Glu Asn Asn
                405                 410                 415
Tyr Lys Thr Thr Pro Pro Val Leu Asp Ser Asp Gly Ser Phe Phe Leu
            420                 425                 430

Tyr Ser Lys Leu Thr Val Asp Lys Ser Arg Trp Gln Gln Gly Asn Val
        435                 440                 445
Phe Ser Cys Ser Val Met His Glu Ala Leu His Asn His Tyr Thr Gln
    450                 455                 460
Lys Ser Leu Ser Leu Ser Pro Gly Lys
465                 470

<210> 11
<211> 235
<212> PRT
<213> Homo sapiens

<400> 11
Met Thr Cys Ser Pro Leu Leu Leu Thr Leu Leu Ile His Cys Thr Gly
  1               5                  10                  15
Ser Trp Ala Gln Ser Val Leu Thr Gln Pro Pro Ser Val Ser Ala Ala
             20                  25                  30
Pro Gly Gln Lys Val Thr Ile Ser Cys Ser Gly Arg Ser Ser Thr Ile
         35                  40                  45
Ala Val Asn Ser Val Ser Trp Tyr Gln Leu Leu Pro Gly Ser Ala Pro
     50                  55                  60
Lys Leu Leu Ile Tyr Asp Thr Thr Lys Arg Pro Ser Gly Ile Pro Asp
 65                  70                  75                  80
Arg Phe Ser Gly Ser Lys Ser Gly Thr Ser Ala Thr Leu Gly Ile Thr
                 85                  90                  95
Gly Leu Arg Thr Gly Asp Glu Ala Val Tyr Tyr Cys Ser Val Val Arg
            100                 105                 110
Tyr Asn Tyr Val Arg Leu Arg Phe Gly Gly Gly Thr His Leu Thr Val
        115                 120                 125
Leu Gly Gln Pro Lys Ala Ala Pro Ser Val Thr Leu Phe Pro Pro Ser
    130                 135                 140
Ser Glu Glu Leu Gln Ala Asn Lys Ala Thr Leu Val Cys Leu Ile Ser
145                 150                 155                 160
Asp Phe Tyr Pro Gly Ala Val Thr Val Ala Trp Lys Ala Asp Ser Ser
                165                 170                 175
Pro Val Lys Ala Gly Val Glu Thr Thr Thr Pro Ser Lys Gln Ser Asn

            180                 185                 190
Asn Lys Tyr Ala Ala Ser Ser Tyr Leu Ser Leu Thr Pro Glu Gln Trp
        195                 200                 205
Lys Ser His Lys Ser Tyr Ser Cys Gln Val Thr His Glu Gly Ser Thr
    210                 215                 220
Val Glu Lys Thr Val Ala Pro Thr Glu Cys Ser
225                 230                 235

<210> 12
<211> 473
<212> PRT
<213> Homo sapiens

<400> 12
Met Asp Trp Thr Trp Arg Phe Leu Phe Val Val Ala Ala Ala Thr Gly
1               5                   10                  15
Val Gln Ser Gln Glu Gln Leu Val Gln Ser Gly Ala Glu Val Lys Gln
            20                  25                  30
Thr Gly Ser Ser Val Arg Val Ser Cys Lys Ala Ser Gly Gly Thr Phe
        35                  40                  45
Ser Arg Tyr Ala Ile Asn Trp Val Arg Gln Val Pro Gly Gln Gly Leu
    50                  55                  60
Glu Trp Met Ala Gly Ile Ile Pro Ile Phe Gly Pro Pro Lys Tyr Ala
65                  70                  75                  80
Gln Lys Phe Gln Gly Arg Val Ser Leu Thr Ala Asp Arg Ser Thr Asn
                85                  90                  95
Thr Ala Tyr Met Glu Met Ala Arg Leu Arg Ser Asp Asp Thr Ala Val
            100                 105                 110
Tyr Tyr Cys Ala Arg Pro Gly Arg Phe Pro Leu Tyr Arg Asp Trp Pro
        115                 120                 125
Gly Phe Asp Tyr Trp Gly Gln Gly Thr Pro Val Ala Val Ser Ser Ala
    130                 135                 140
Ser Thr Lys Gly Pro Ser Val Phe Pro Leu Ala Pro Ser Ser Lys Ser
145                 150                 155                 160
Thr Ser Gly Gly Thr Ala Ala Leu Gly Cys Leu Val Lys Asp Tyr Phe
                165                 170                 175

Pro Glu Pro Val Thr Val Ser Trp Asn Ser Gly Ala Leu Thr Ser Gly
            180                 185                 190
Val His Thr Phe Pro Ala Val Leu Gln Ser Ser Gly Leu Tyr Ser Leu
        195                 200                 205
Ser Ser Val Val Thr Val Pro Ser Ser Ser Leu Gly Thr Gln Thr Tyr
    210                 215                 220
Ile Cys Asn Val Asn His Lys Pro Ser Asn Thr Lys Val Asp Lys Lys
225                 230                 235                 240
Val Glu Pro Lys Ser Cys Asp Lys Thr His Thr Cys Pro Pro Cys Pro
                245                 250                 255
Ala Pro Glu Leu Leu Gly Gly Pro Ser Val Phe Leu Phe Pro Pro Lys
            260                 265                 270
Pro Lys Asp Thr Leu Met Ile Ser Arg Thr Pro Glu Val Thr Cys Val
        275                 280                 285
Val Val Asp Val Ser His Glu Asp Pro Glu Val Lys Phe Asn Trp Tyr
    290                 295                 300
Val Asp Gly Val Glu Val His Asn Ala Lys Thr Lys Pro Arg Glu Glu
305                 310                 315                 320
Gln Tyr Asn Ser Thr Tyr Arg Val Val Ser Val Leu Thr Val Leu His
                325                 330                 335
Gln Asp Trp Leu Asn Gly Lys Glu Tyr Lys Cys Lys Val Ser Asn Lys
            340                 345                 350
Ala Leu Pro Ala Pro Ile Glu Lys Thr Ile Ser Lys Ala Lys Gly Gln
        355                 360                 365
Pro Arg Glu Pro Gln Val Tyr Thr Leu Pro Pro Ser Arg Asp Glu Leu
    370                 375                 380
Thr Lys Asn Gln Val Ser Leu Thr Cys Leu Val Lys Gly Phe Tyr Pro
385                 390                 395                 400
Ser Asp Ile Ala Val Glu Trp Glu Ser Asn Gly Gln Pro Glu Asn Asn
                405                 410                 415
Tyr Lys Thr Thr Pro Pro Val Leu Asp Ser Asp Gly Ser Phe Phe Leu
            420                 425                 430
Tyr Ser Lys Leu Thr Val Asp Lys Ser Arg Trp Gln Gln Gly Asn Val
        435                 440                 445
Phe Ser Cys Ser Val Met His Glu Ala Leu His Asn His Tyr Thr Gln
    450                 455                 460

Lys Ser Leu Ser Leu Ser Pro Gly Lys
465                 470

<210> 13
<211> 235
<212> PRT
<213> Homo sapiens

<400> 13
Met Thr Cys Ser Pro Leu Leu Leu Thr Leu Leu Ile His Cys Thr Gly
1               5                   10                  15
Ser Trp Ala Gln Ser Val Leu Thr Gln Pro Pro Ser Val Ser Ala Ala
            20                  25                  30
Pro Gly Gln Lys Val Thr Ile Ser Cys Ser Gly Arg Ser Ser Thr Ile
        35                  40                  45
Ala Val Asn Ser Val Ser Trp Tyr Gln Leu Leu Pro Gly Ser Ala Pro
    50                  55                  60
Lys Leu Leu Ile Tyr Asp Thr Thr Lys Arg Pro Ser Gly Ile Pro Asp
65                  70                  75                  80
Arg Phe Ser Gly Ser Lys Ser Gly Thr Ser Ala Thr Leu Gly Ile Thr
                85                  90                  95
Gly Leu Arg Thr Gly Asp Glu Ala Val Tyr Tyr Cys Gly Ser Gly Arg
            100                 105                 110
Ser Val Ala Asn Pro Pro Val Phe Gly Gly Gly Thr His Leu Thr Val
        115                 120                 125
Leu Gly Gln Pro Lys Ala Ala Pro Ser Val Thr Leu Phe Pro Pro Ser
    130                 135                 140
Ser Glu Glu Leu Gln Ala Asn Lys Ala Thr Leu Val Cys Leu Ile Ser
145                 150                 155                 160
Asp Phe Tyr Pro Gly Ala Val Thr Val Ala Trp Lys Ala Asp Ser Ser
                165                 170                 175
Pro Val Lys Ala Gly Val Glu Thr Thr Thr Pro Ser Lys Gln Ser Asn
            180                 185                 190
Asn Lys Tyr Ala Ala Ser Ser Tyr Leu Ser Leu Thr Pro Glu Gln Trp
        195                 200                 205
Lys Ser His Lys Ser Tyr Ser Cys Gln Val Thr His Glu Gly Ser Thr

210                 215                 220
Val Glu Lys Thr Val Ala Pro Thr Glu Cys Ser
225                 230                 235

<210> 14
<211> 473
<212> PRT
<213> Homo sapiens

<400> 14
Met Asp Trp Thr Trp Arg Phe Leu Phe Val Val Ala Ala Ala Thr Gly
1               5                   10                  15
Val Gln Ser Gln Glu Gln Leu Val Gln Ser Gly Ala Glu Val Lys Gln
            20                  25                  30
Thr Gly Ser Ser Val Arg Val Ser Cys Lys Ala Ser Gly Gly Thr Phe
        35                  40                  45
Ser Arg Tyr Ala Ile Asn Trp Val Arg Gln Val Pro Gly Gln Gly Leu
    50                  55                  60
Glu Trp Met Ala Gly Ile Ile Pro Ile Phe Gly Pro Pro Lys Tyr Ala
65                  70                  75                  80
Gln Lys Phe Gln Gly Arg Val Ser Leu Thr Ala Asp Arg Ser Thr Asn
                85                  90                  95
Thr Ala Tyr Met Glu Met Ala Arg Leu Arg Ser Asp Asp Thr Ala Val
            100                 105                 110
Tyr Tyr Cys Ala Arg Ala Pro Thr Pro Leu Tyr Asp Thr Glu Thr Gly
        115                 120                 125
Pro Phe Asp Tyr Trp Gly Gln Gly Thr Pro Val Ala Val Ser Ser Ala
    130                 135                 140
Ser Thr Lys Gly Pro Ser Val Phe Pro Leu Ala Pro Ser Ser Lys Ser
145                 150                 155                 160
Thr Ser Gly Gly Thr Ala Ala Leu Gly Cys Leu Val Lys Asp Tyr Phe
                165                 170                 175
Pro Glu Pro Val Thr Val Ser Trp Asn Ser Gly Ala Leu Thr Ser Gly
            180                 185                 190
Val His Thr Phe Pro Ala Val Leu Gln Ser Ser Gly Leu Tyr Ser Leu
        195                 200                 205

Ser Ser Val Val Thr Val Pro Ser Ser Ser Leu Gly Thr Gln Thr Tyr
    210                 215                 220
Ile Cys Asn Val Asn His Lys Pro Ser Asn Thr Lys Val Asp Lys Lys
225                 230                 235                 240
Val Glu Pro Lys Ser Cys Asp Lys Thr His Thr Cys Pro Pro Cys Pro
                245                 250                 255
Ala Pro Glu Leu Leu Gly Gly Pro Ser Val Phe Leu Phe Pro Pro Lys
            260                 265                 270
Pro Lys Asp Thr Leu Met Ile Ser Arg Thr Pro Glu Val Thr Cys Val
        275                 280                 285
Val Val Asp Val Ser His Glu Asp Pro Glu Val Lys Phe Asn Trp Tyr
    290                 295                 300
Val Asp Gly Val Glu Val His Asn Ala Lys Thr Lys Pro Arg Glu Glu
305                 310                 315                 320
Gln Tyr Asn Ser Thr Tyr Arg Val Val Ser Val Leu Thr Val Leu His
                325                 330                 335
Gln Asp Trp Leu Asn Gly Lys Glu Tyr Lys Cys Lys Val Ser Asn Lys
            340                 345                 350
Ala Leu Pro Ala Pro Ile Glu Lys Thr Ile Ser Lys Ala Lys Gly Gln
        355                 360                 365
Pro Arg Glu Pro Gln Val Tyr Thr Leu Pro Pro Ser Arg Asp Glu Leu
    370                 375                 380
Thr Lys Asn Gln Val Ser Leu Thr Cys Leu Val Lys Gly Phe Tyr Pro
385                 390                 395                 400
Ser Asp Ile Ala Val Glu Trp Glu Ser Asn Gly Gln Pro Glu Asn Asn
                405                 410                 415
Tyr Lys Thr Thr Pro Pro Val Leu Asp Ser Asp Gly Ser Phe Phe Leu
            420                 425                 430
Tyr Ser Lys Leu Thr Val Asp Lys Ser Arg Trp Gln Gln Gly Asn Val
        435                 440                 445
Phe Ser Cys Ser Val Met His Glu Ala Leu His Asn His Tyr Thr Gln
    450                 455                 460
Lys Ser Leu Ser Leu Ser Pro Gly Lys
465                 470

<210> 15

<211> 235
<212> PRT
<213> Homo sapiens

<400> 15
Met Thr Cys Ser Pro Leu Leu Leu Thr Leu Leu Ile His Cys Thr Gly
1               5                   10                  15
Ser Trp Ala Gln Ser Val Leu Thr Gln Pro Pro Ser Val Ser Ala Ala
            20                  25                  30
Pro Gly Gln Lys Val Thr Ile Ser Cys Ser Gly Arg Ser Ser Thr Ile
        35                  40                  45
Ala Val Asn Ser Val Ser Trp Tyr Gln Leu Leu Pro Gly Ser Ala Pro
    50                  55                  60
Lys Leu Leu Ile Tyr Asp Thr Thr Lys Arg Pro Ser Gly Ile Pro Asp
65                  70                  75                  80
Arg Phe Ser Gly Ser Lys Ser Gly Thr Ser Ala Thr Leu Gly Ile Thr
                85                  90                  95
Gly Leu Arg Thr Gly Asp Glu Ala Val Tyr Tyr Cys Ala Leu Leu Glu
            100                 105                 110
Glu Gly Ala Lys Gly Pro Glu Phe Gly Gly Gly Thr His Leu Thr Val
        115                 120                 125
Leu Gly Gln Pro Lys Ala Ala Pro Ser Val Thr Leu Phe Pro Pro Ser
    130                 135                 140
Ser Glu Glu Leu Gln Ala Asn Lys Ala Thr Leu Val Cys Leu Ile Ser
145                 150                 155                 160
Asp Phe Tyr Pro Gly Ala Val Thr Val Ala Trp Lys Ala Asp Ser Ser
                165                 170                 175
Pro Val Lys Ala Gly Val Glu Thr Thr Thr Pro Ser Lys Gln Ser Asn
            180                 185                 190
Asn Lys Tyr Ala Ala Ser Ser Tyr Leu Ser Leu Thr Pro Glu Gln Trp
        195                 200                 205
Lys Ser His Lys Ser Tyr Ser Cys Gln Val Thr His Glu Gly Ser Thr
    210                 215                 220
Val Glu Lys Thr Val Ala Pro Thr Glu Cys Ser
225                 230                 235

<210> 16
<211> 473
<212> PRT
<213> Homo sapiens

<400> 16
Met Asp Trp Thr Trp Arg Phe Leu Phe Val Val Ala Ala Ala Thr Gly
1               5                   10                  15
Val Gln Ser Gln Glu Gln Leu Val Gln Ser Gly Ala Glu Val Lys Gln
            20                  25                  30
Thr Gly Ser Ser Val Arg Val Ser Cys Lys Ala Ser Gly Gly Thr Phe
        35                  40                  45
Ser Arg Tyr Ala Ile Asn Trp Val Arg Gln Val Pro Gly Gln Gly Leu
    50                  55                  60
Glu Trp Met Ala Gly Ile Ile Pro Ile Phe Gly Pro Pro Lys Tyr Ala
65                  70                  75                  80
Gln Lys Phe Gln Gly Arg Val Ser Leu Thr Ala Asp Arg Ser Thr Asn
                85                  90                  95
Thr Ala Tyr Met Glu Met Ala Arg Leu Arg Ser Asp Asp Thr Ala Val
            100                 105                 110
Tyr Tyr Cys Ala Arg Pro Val Pro Ala Thr Tyr Glu Ser Thr Ala Gly
        115                 120                 125
Tyr Phe Asp Tyr Trp Gly Gln Gly Thr Pro Val Ala Val Ser Ser Ala
    130                 135                 140
Ser Thr Lys Gly Pro Ser Val Phe Pro Leu Ala Pro Ser Ser Lys Ser
145                 150                 155                 160
Thr Ser Gly Gly Thr Ala Ala Leu Gly Cys Leu Val Lys Asp Tyr Phe
                165                 170                 175
Pro Glu Pro Val Thr Val Ser Trp Asn Ser Gly Ala Leu Thr Ser Gly
            180                 185                 190
Val His Thr Phe Pro Ala Val Leu Gln Ser Ser Gly Leu Tyr Ser Leu
        195                 200                 205
Ser Ser Val Val Thr Val Pro Ser Ser Ser Leu Gly Thr Gln Thr Tyr
    210                 215                 220
Ile Cys Asn Val Asn His Lys Pro Ser Asn Thr Lys Val Asp Lys Lys
225                 230                 235                 240

Val Glu Pro Lys Ser Cys Asp Lys Thr His Thr Cys Pro Pro Cys Pro
                245                 250                 255
Ala Pro Glu Leu Leu Gly Gly Pro Ser Val Phe Leu Phe Pro Pro Lys
            260                 265                 270
Pro Lys Asp Thr Leu Met Ile Ser Arg Thr Pro Glu Val Thr Cys Val
        275                 280                 285
Val Val Asp Val Ser His Glu Asp Pro Glu Val Lys Phe Asn Trp Tyr
    290                 295                 300
Val Asp Gly Val Glu Val His Asn Ala Lys Thr Lys Pro Arg Glu Glu
305                 310                 315                 320
Gln Tyr Asn Ser Thr Tyr Arg Val Val Ser Val Leu Thr Val Leu His
                325                 330                 335
Gln Asp Trp Leu Asn Gly Lys Glu Tyr Lys Cys Lys Val Ser Asn Lys
            340                 345                 350
Ala Leu Pro Ala Pro Ile Glu Lys Thr Ile Ser Lys Ala Lys Gly Gln
        355                 360                 365
Pro Arg Glu Pro Gln Val Tyr Thr Leu Pro Pro Ser Arg Asp Glu Leu
    370                 375                 380
Thr Lys Asn Gln Val Ser Leu Thr Cys Leu Val Lys Gly Phe Tyr Pro
385                 390                 395                 400
Ser Asp Ile Ala Val Glu Trp Glu Ser Asn Gly Gln Pro Glu Asn Asn
                405                 410                 415
Tyr Lys Thr Thr Pro Pro Val Leu Asp Ser Asp Gly Ser Phe Phe Leu
            420                 425                 430
Tyr Ser Lys Leu Thr Val Asp Lys Ser Arg Trp Gln Gln Gly Asn Val
        435                 440                 445
Phe Ser Cys Ser Val Met His Glu Ala Leu His Asn His Tyr Thr Gln
    450                 455                 460
Lys Ser Leu Ser Leu Ser Pro Gly Lys
465                 470

<210> 17
<211> 235
<212> PRT
<213> Homo sapiens

<400> 17
Met Thr Cys Ser Pro Leu Leu Leu Thr Leu Leu Ile His Cys Thr Gly
1               5                   10                  15
Ser Trp Ala Gln Ser Val Leu Thr Gln Pro Pro Ser Val Ser Ala Ala
            20                  25                  30
Pro Gly Gln Lys Val Thr Ile Ser Cys Ser Gly Arg Ser Ser Thr Ile
        35                  40                  45
Ala Val Asn Ser Val Ser Trp Tyr Gln Leu Leu Pro Gly Ser Ala Pro
    50                  55                  60
Lys Leu Leu Ile Tyr Asp Thr Thr Lys Arg Pro Ser Gly Ile Pro Asp
65                  70                  75                  80
Arg Phe Ser Gly Ser Lys Ser Gly Thr Ser Ala Thr Leu Gly Ile Thr
                85                  90                  95
Gly Leu Arg Thr Gly Asp Glu Ala Val Tyr Tyr Cys Ser Pro Val Gln
            100                 105                 110
Arg Tyr Ser Gly Gly Ser Leu Phe Gly Gly Gly Thr His Leu Thr Val
        115                 120                 125
Leu Gly Gln Pro Lys Ala Ala Pro Ser Val Thr Leu Phe Pro Pro Ser
    130                 135                 140
Ser Glu Glu Leu Gln Ala Asn Lys Ala Thr Leu Val Cys Leu Ile Ser
145                 150                 155                 160
Asp Phe Tyr Pro Gly Ala Val Thr Val Ala Trp Lys Ala Asp Ser Ser
                165                 170                 175
Pro Val Lys Ala Gly Val Glu Thr Thr Thr Pro Ser Lys Gln Ser Asn
            180                 185                 190
Asn Lys Tyr Ala Ala Ser Ser Tyr Leu Ser Leu Thr Pro Glu Gln Trp
        195                 200                 205
Lys Ser His Lys Ser Tyr Ser Cys Gln Val Thr His Glu Gly Ser Thr
    210                 215                 220
Val Glu Lys Thr Val Ala Pro Thr Glu Cys Ser
225                 230                 235

<210> 18
<211> 29
<212> DNA
<213> Artificial Sequence

<220>
<223> Description of Artificial Sequence:Synthesized DNA

<400> 18
agcgtcgaca tggactggac ctggaggtt 29

<210> 19
<211> 24
<212> DNA
<213> Artificial Sequence

<220>
<223> Description of Artificial Sequence:Synthesized DNA

<400> 19
agccggaggt ggttcccggg tagc 24

<210> 20
<211> 30
<212> DNA
<213> Artificial Sequence

<220>
<223> Description of Artificial Sequence:Synthesized DNA

<400> 20
cctccaccaa gggcccatcg gtcttccccc 30

<210> 21
<211> 33
<212> DNA
<213> Artificial Sequence

<220>
<223> Description of Artificial Sequence:Synthesized DNA

<400> 21
gagggacaga ggcccattta ctcgccggcg att 33

<210> 22
<211> 29
<213> Artificial Sequence

<220>
<223> Description of Artificial Sequence:Synthesized DNA

<400> 22
   agcgtcgaca tgacctgctc ccctctcct 29

<210> 23
<211> 29
<212> DNA
<213> Artificial Sequence

<220>
<223> Description of Artificial Sequence:Synthesized DNA

<400> 23
ggatgtctta cgagtatccg ccggcgatt 29

## Description

### FIELD OF THE INVENTION

The present invention relates to a novel peptide, a method of screening using the peptide, and an antibody obtained by the method of screening. More particularly, the present invention relates to a novel peptide which binds to a human-type antihuman IgE monoclonal antibody, a method of screening the human-type antihuman IgE monoclonal antibody using the peptide, and the human-type antihuman IgE monoclonal antibody obtained by the method of screening.

Furthermore, the present invention relates to a medicine for preventing and/or treating allergy diseases comprising the human-type antihuman IgE monoclonal antibody as an effective component.

### DESCRIPTION OF BACKGROUND ART

Conventionally, antibodies have been applied not only for the determination of antigens and diagnosis of diseases, and the like, but also for treating diseases. However, the application of antibodies as a remedy for diseases has been limited until recently because antibodies have polyclonal properties and because of other reasons . Thus , discovery of a method of producing monoclonal antibodies (MAb) exhibiting a specificity (Koehler and Milstein, Nature, (1975) 265, 295-497) has made the possibility of using antibodies as a remedy more realistic.

Many MAbs are mouse proteins because they are produced from hybridomas obtained by cell fusion of mouse spleen cells and mouse myeloma cells. However, there have been only a few reports relating to production of MAb derived from human cells. Since most available MAbs are derived from mice, they are antigenic to humans. Therefore, if administered to humans, these MAbs induce an undesirable immune response, called a human anti-mouse antibody (HAMA) reaction. Specifically, humans exhibit an antibody producing response to a mouse MAb, completely exclude the mouse MAb, or reduce the effect of the mouse MAb. Therefore, the use of mouse MAb as a remedy for humans is substantially limited. In practice, MAb derived from mouse cannot be usable for treating diseases more than several times. For these reasons, producing a non-human antibody with reduced antigenic properties to humans has been proposed. Such a technology is generally called "hominization". A DNA recombination technique to manipulate a DNA sequence encoding polypeptide chains of antibody molecules can be used in this technology. The method of hominizing MAb in the early days was by producing a chimeric antibody composed of a whole variable region of mouse antibody fused with a constant region of human antibody. European Patent No . 0120694 (Celltech), European Patent No. 0012023 (Genentech), European Patent Publication No. 0171496 (Japanese Patent Application Laid-open No. 169370/1984, Research Development Corporation of Japan), European Patent Publication No. 0173494 (Stanford University), and European Patent Publication No. 0194276 (Celltech) report methods of chimeraplasty manipulation. Thereafter, a number of patent applications relating to chimeric antibodies, including a tumor specific chimeric antibody (European Patent No. 0256654 to Centocor, Inc.), an anticarcinogenic fetal antigen chimeric antibody (European Patent Publication No. 0125023, Genentech, Inc.; European Patent No. 0332424, Hybritech, Inc.), and the like have been published.

However, because such a hominized chimeric antibody still contains significant nonhuman amino acid sequences, i.e. variable regions originating from mice, the hominized antibody may induce a certain kind of HAMA reaction especially when administered over a long period of time (Begent, et al., Br. J. Cancer, (1990) 62,487). Winter reports a method of transplanting a complementarity determining region (CDR) of mouse MAb into a frame work (FR) region of a human immunoglobulin variable region by site specific mutagenesis using a long oligonucleotide (European Patent Publication No. 0239400). The report also mentions the possibility of altering the amino acid sequence in the FR region. There is a report on the preparation of human MAb by CDR transplantation from a mouse MAb capable of recognizing lysozyme and a mouse MAb capable of recognizing an antigen on human T-cells (Verhoeyen, et al., Science, (1988) 239, 1534 ; Riechmann, et al., Nature, (1988) 332, 323) . Such a CDR transplant hominized antibody may induce fewer HAMA responses than a chimeric antibody because of a smaller proportion of nonhuman amino acid sequences contained therein.

In the application of an antibody as a diagnostic agent or therapeutic agent for a disease, antibodies to be used are required to have a high antigen affinity (binding activity) for the construction of a highly sensitive measurement system or effective remedy for the disease. However, Riechmann et al. indicated that independent CDR importation is insufficient to give an antigen binding activity satisfactory for a CDR-transplanted antibody, and that amino acid residues in the FR region adjoining the CDR region must be altered to maintain the antigen binding activity. Affinity of most CDR transplant antibodies prepared is significantly lower than that of original mouse MAb. Queen et al. have reported preparation of an antibody binding with interleukin 2 receptor by transplanting CDR of mouse MAb (anti-Tac antibody) into FR of the human immunoglobulin variable region and by binding the resulting product with a constant region (Queen, et al., Proc. Natl. Acad. Sci., USA (1989) 86,10029 and WO90/07861). The human FR region was selected so as to maximize homology with the sequence of mouse anti-Tac antibody. In addition, FR amino acid residues which easily interact with the CDR or antigen were identified using computer modeling, and amino acids originating from mouse antibody were replaced at these sites in the human antibody. However, the anti-Tac antibody altered in this manner possessed only about 1/3 of the affinity of the mouse-type antibody. As can be seen from this example, a CDR transplant antibody with a satisfactory affinity cannot be easily prepared. Specifically, simply transplanting CDR of a donor antibody into FR of the acceptor antibody is not sufficient. To maintain affinity, it is necessary to change residues in FR of the acceptor antibody. However, at the present time it is impossible to anticipate which residues should be changed based on available prior art.

On the other hand, as a method of preparing a human antibody, a method of preparing human MAb binding with specific antigens by applying a hybridoma method, which is conventionally used for the preparation of mouse MAb, has been reported. However, there are only a few successful examples . For example, Reports by Carrol, W.L. et al. (Carrol, W.L., et al., J. Immunol. Methods, (1986) 89, 61), and Strike, L. E et al. (Strike, L.E. , et al., J. Immunol., (1984) 132,1788) can'be given. Under this situation, various improvements to establish human hybridoma cells may have advanced the possibility of preparation of human-type monoclonal antibodies. However, antigens recognized by human-type antibodies which have been obtained heretofore are foreign heterologous proteins, and it is almost impossible to recognize human origin proteins. In addition, according to an *invitro* sensitization method, antibodies exhibiting only a low affinity with antigens can be obtained at the present time. Specifically, preparing a human-type monoclonal antibody is not as easy as preparing a mouse MAb at the present time. Further improvements in the preparation method are desired. The present inventors have established a method of acquiring a human-type antihuman IgE monoclonal autoantibody with high specificity and have succeeded in preparing a recombinant antibody by using a genetic engineering technique. The recombinant human antihuman IgE antibody prepared using said method is expected to be useful for the remedy of I-type allergy. An allergy is a morbid state induced by a sensitive immune response to an allergen and includes an immediate type (I-type) allergy characterized by an allergic reaction occurring immediately after invasion of an allergen into the body and a delayed type hypersensitivity based on a cellular immunity reaction mediated by T-cells. In recent years, the term "allergy" may be used to denote I-type hypersensitivity. The I-type allergy is induced by an allergen-specific IgE antibody produced by B-cells (plasma cells) which are differentiated by allergenic stimulus. The secreted IgE antibody combines with an Fcε receptor (FcεR) existing in basophiles circulating in the body and cell membrane of mast cells which are present in the tissues. If an allergen combines with an IgE bonded with a receptor, cross-linking of FcεR via the allergen occurs. As a result a degranulation reaction from the cells induced, in which various chemical transmitters, such as histamine and prostaglandin, and chemical mediators such as leukotriene, and the like are released. These substances are known as causal substances causing typical clinical symptoms of I-type allergy.

There are two major approaches for treating an allergy at the present time. One is a symptomatic treatment of allergy symptoms by using a medicine which obstructs a chemical mediator, for example, an antihistaminic agent, or by using a cell proliferation inhibitor or an immunosuppressor such as corticosteroid. This method requires repeated administration and often is accompanied by undesirable side effects. Another treatment method is the desensitization treatment, in which the allergen causing the disease is administered for a prescribed period of time so that the patient can acquire an immune tolerance against the specific allergen. However, because the specific allergen cannot always be identified, the treatment often brings about no effect. Furthermore, the patients may suffer from considerable pain and an unpleasant feeling. The treatment method itself cannot always be without risk. Neither approach can prevent occurrence of an anaphylaxia symptom.

Since the I-type allergy is known to be induced due to the increase of IgE, a trial for the prevention or cure of the I-type allergy using a substance, particularly an antibody, which obstructs IgE production during an early stage inducing allergic reaction and bonding of IgE with basophiles or mast cells, has been undertaken. For example, Japanese Patent Application Laid-open Nos. 289100/1986 and No. 127977/1991 disclose prevention or treatment by obstructing bonding of IgE with basophiles or mast cells using an antibody to FcεR. Furthermore, concerning prevention or treatment of an allergy using an antibody to IgE, a method of controlling an IgE antibody-producing response (Japanese Patent Application Laid-open No. 72500/1991) and a method for treating by removing IgE production cells by selectively injuring IgE-producing B-cells using an anti-IgE antibody which is combined with a cytotoxin (Japanese Patent Application Laid-open Nos. 102032/1989 and 501927/1991) have been disclosed. However, antihuman IgE antibodies used in these examples are antibodies of goat or mouse origin or mouse-human chimeric antibodies . Antibodies derived from animals other than human exhibit a significantly shorter blood half-life than human antibodies in the treatment. In addition, since heteroantibodies produce an antibody against the antibody due to the possession of antigenicity, the therapeutic effect decreases or disappears. For these reasons, the antihuman IgE antibody used for treating allergy is most preferably a human-type antibody. Furthermore, the anti-IgE antibody applicable for treatment of an allergy needs to recognize the Fc area of IgE to prevent side effects and possess a high affinity with IgE at the same time, by the reason that IgE is present only in a slight amount in serum.

### DISCLOSURE OF THE INVENTION

In order to provide a high affinity human MAb recognizing human IgE and possibly being a basic allergy curing medicine, a human-type antibody needs to provide following features;
(1) possessing specificity to the human IgE,
(2) exhibiting high affinity to the human IgE,
(3) not releasing a chemical mediator from cells having FcεRI, and
(4) selectively combining with human IgE-producing cells.

The human-type antibody having these characteristics is thought to bind to secreted IgE in blood, thereby inhibiting binding of the IgE to FcεR. Furthermore, the substance resulting from the binding of the antibody to a cell proliferation inhibitor or cytotoxic substance is thought to effectively injure the IgE-producing cells, thereby controlling production of the IgE. Therefore, antibodies possessing these characteristics are expected to be very useful for the basic treatment and prevention of allergies.

Thus, the inventors of the present invention have carried out extensive studies to discover a human-type antihuman IgE monoclonal antibody exhibiting minimal side effects and capable of specifically combining with IgE. As a result, the inventors have found a novel peptide comprising 18 amino acids which can be used as a tool for acquiring such an antibody. The inventors have further found that a human-type anti-IgE monoclonal antibody possessing the above-described characteristics can be obtained using said peptide. Accordingly, an object of the present invention is to provide a novel peptide recognized by a human-type antihuman IgE monoclonal antibody, a method of screening the human-type antihuman IgE monoclonal antibody using the peptide, the human-type antihuman IgE monoclonal antibody obtained by the method of screening, and a remedy for allergic diseases using the monoclonal antibody.

The present invention relates to a novel peptide and, more particularly, to a novel peptide binding to a human-type antihuman IgE monoclonal antibody. The novel peptide of the present invention is useful as a tool for screening a human-type antihuman IgE monoclonal antibody possessing high specificity.

The present invention also relates to a screening method using the peptide and, more particularly, to a method of screening a human-type antihuman IgE monoclonal antibody using the peptide . According to the screening method of the present invention, a human-type antihuman IgE monoclonal antibody possessing high specificity can be efficiently obtained.

The present invention further relates to an antibody obtained by the screening method and, more particularly, to a human-type antihuman IgE monoclonal antibody obtained by the screening method.

Furthermore, the present invention relates to a pharmaceutical for preventing or treating allergic diseases comprising the antibody thus obtained as an effective component. The peptide of the present invention can be used together with other adjuvants as a vaccine for preventing or treating allergic diseases.

The novel peptide of the present invention (hereinafter referred to as "peptide of the present invention") is a peptide designed based on a human IgE (Elvin A. Kabat, et al., "SEQUENCES OF PROTEINS OF IMMUNOLOGICAL INTEREST" Vol. 1, FIFTH EDITION (1991), published by U.S. DEPARTMENT OF HEALTH AND HUMAN SERVICES). This is a partial peptide (F349-A369) containing 18 amino acids derived from the CH2-CH3 region of human IgE and includes the sequence shown as Sequence ID No. 1 in the Sequence Table. The antibody specific to the peptide sequence of the present invention can recognize any IgEs such as a soluble IgE (secretion-type IgE) and an IgE existing on the surface of IgE-producing cells (membrane binding-type IgE). The peptide of the present invention can be easily synthesized using a commercially available peptide synthesis apparatus. For example, the peptide can be synthesized using a peptide synthesis apparatus (Model 431A, manufactured by Applied Biosystems Inc.). Specifically, a dipeptide is synthesized by adsorbing amino acids with protected active groups in a resin, adding a deblocking solution into the resin to remove the protection groups, adding amino acids with protected active groups, and reacting these amino acids. This procedure is repeated to synthesize a peptide having the target sequence. The protecting groups of the peptide can be removed by reacting in a removing solution (Applied Biosystems Inc.) for several hours. The peptide can be collected as a precipitate by adding diethyl ether to the resulting filtrate. This peptide is dissolved in 2N acetic acid solution and lyophilized to obtain a crude peptide. The crude peptide thus obtained can be purified by reverse phase chromatography and the like. The objective peptide has a specific elution site. The amino acid sequence of the obtained peptide can be determined by sequence analysis using a protein sequencer and amino acid analysis using an amino acid analyzer. This peptide is characterized by having a high affinity to the antibody which possesses an affinity to Fcε of IgE antibody. The peptide of the present invention can be used as a vaccine for preventing and/or treating allergic diseases.

A human-type antihuman IgE monoclonal antibody can be screened using the peptide of the present invention. Specifically, to prepare a human antibody to the IgE, autoantibody producing cells are established from human lymphocyte cells sensitized *in vitro* by a hybridoma method. A human-type antihuman IgE monoclonal antibody with high specificity having the above-described characteristics desired for a remedy for allergic diseases can be obtained by using the affinity to the peptide of the present invention as an index from the human-type antihuman IgE monoclonal antibody produced by the established cells. The binding activity to the peptide of the present invention can be determined by preparing a plate on which the peptide of the present invention has been immobilized, adding a supernatant of cultured medium or the like of the antibody-producing cells, and using the EIA Method or RIA method. Cells producing the human-type antihuman IgE monoclonal antibody with high specificity to human IgE having the above-described characteristics desired for a remedy for allergic diseases can be selected using this method.

A gene encoding an antibody variable region (VH and VL) is cloned from the cells selected in this manner. Using DNA encoding this antibody variable region, a human-type antihuman IgE monoclonal antibody can be prepared as a recombinant protein by means of a genetic engineering technique. Furthermore, as required, for the purpose of producing an antibody exhibiting higher affinity, a random mutagenesis is introduced into the CDR area of the VH and VL regions using a gene manipulation technique. After preparation of an antibody DNA library, the antibody genes are caused to express, and phage antibodies exhibiting a high affinity are selected using a panning method and the like, whereby the human-type antihuman IgE monoclonal antibody exhibiting a high affinity can be prepared.

The human-type antihuman IgE monoclonal antibody of the present invention can be prepared as follows.

### (1) Antigen

Preferably purified IgE of human origin is used as an immunogen. Said purified IgE can be obtained by purifying blood serum of healthy persons or myeloma patients, or by purifying a supernatant of cultured medium of human IgE-producing cells.

### (2) preparation of hybridoma cells

Human lymphocytes are sensitized *in vitro* using human IgE as an immunogen. The sensitized lymphocytes are fused with myeloma cells using polyethylene glycol. As myeloma cells, human origin LICR-LON-HMy2, WI-L2/729HF2, 8226AR/NIP4-1, and K6H6/B5 can be used.

### (3) Selection of hybridoma cells

Hybridoma cells producing a human MAb antibody can be selected using a denovo DNA replication inhibitor. When the K6H6/B5 cell described in the examples of the present invention is used as a myeloma cell, a medium containing a zaserine is employed for the selection. In addition, only the cells producing the objective antibody can be selected as hybridoma cells by analyzing the supernatant of cultured medium. The ELISA method, the binding reaction to IgE-producing cells, and the like can be used for the analysis of the supernatant of cultured medium.

### (4) Culturing of hybridoma cells

The selected hybridoma cells can be cultured using RPMI-1640 medium containing fetal bovine serum or a serum-free medium. Microorganisms such as *Escherichia coli* or animal cells such as CHO cells into which the antibody gene has been introduced can also be cultured in accordance with a conventional method. The antibody can be purified from such microorganism cells or supernatant of cultured medium.

### (5) Purification of antibody

The antibody in the resulting supernatant of cultured medium is purified according to a conventional method. For example, ammonium sulfate fractionation, gel filtration, ion exchange chromatography, affinity chromatography, hydrophobic chromatography, and the like can be used either individually or by suitably combining two or more of these methods if necessary.

### (6) Verification of antibody characteristics

The human-type antihuman IgE monoclonal antibody of the present invention not only exhibits a high affinity to the peptide of the present invention, but also possesses the following characteristics:
(i) possesses specificity to the human IgE,
(ii) exhibits a high affinity to IgE,
(iii) does not induce release of a chemical mediator from cells having FcεRI, and
(iv) selectively binds with human IgE-producing cells.

The human-type antihuman IgE monoclonal antibody of the present invention can bind to secreted IgE in blood and inhibit the IgE from binding to FcεR. In addition, the substance resulting from the bonding of the antibody with a cell proliferation inhibitor or cytotoxic substance can effectively injure the IgE-producing cells and thereby control IgE production.

The characteristics of the human-type antihuman IgE monoclonal antibody of the present invention can be confirmed by the following methods.

### (a) Affinity to IgE and specificity

The affinity (i) with the IgE and specificity (ii) of said antibody can be confirmed, for example, by the ELISA method using a membrane on which the human IgE is immobilized or a micro-plate on which the peptide of human IgE origin is immobilized, or by competitive inhibition using human IgE. Bonding with the IgE-producing cells (iv) can be verified by reacting the antibody with human IgE-producing myeloma cells (for example, SKO-007, U266B1, etc.) and detecting the antibodies bound with the cells by the ELISA method or a flow cytometric method. The release of a chemical mediator induced by an antibody treatment (iii) can be confirmed by adding the antibody to peripheral blood mononuclear cells sensitized with an IgE, incubating the mixture, and quantitatively determining chemical transmitters such as histamine discharged from the cells by the RIA method or ELISA method.

### (b) IgE production controlling activity

Since the human MAb alone or the substance resulting from the bonding of the human MAb with a cell proliferation inhibitor or cytotoxic substance is expected to control IgE production by selectively injuring the human IgE-producing cells, there is a possibility of these substances being used as a remedy for allergic diseases. As suitable cell proliferation inhibitors, alkylating agents such as a cyclophosphamide, antimetabolites such as a methotrexate and fluorouracil, antibiotics such as doxorubicin, and the like can be given. As cytotoxic substances, a cytotoxin (ricin and diphteria toxin, for example) and its A-chain can be given. The IgE production controlling activity can be confirmed by culturing the target IgE-producing cells either in the absence or presence of a substance combined with a growth inhibitor or a toxic substance and determining the number of remainning living cells, or by quantitative determination of the amount of IgE production by the ELISA method.

As a human-type antihuman IgE monoclonal antibody with a high specificity thus obtained, the antibodies disclosed in the present invention, specifically the antibodies shown by the Sequence ID No. 10 (H-chain) and No . 11 (L-chain) in the Sequence Table, can be given. These antibodies are useful as a pharmaceutical composition for preventing or treating allergic diseases, a reagent for establishing immunological diagnosis, and the like.

The human-type antihuman IgE monoclonal antibody of the present invention can be administered safely to humans and primates. The protein of the present invention can be made into a preparation and administered either orally or parenterally. Examples of the pharmaceutical preparation include compositions for injection, drip infusion, suppositories, nasal agents, sublingual agents, percutaneous absorption agents, and the like. These preparation are formulated according to known pharmaceutical preparation methods using pharmaceutically acceptable carriers, vehicles, stabilizers, coloring agents, surfactants, and other additives, and made into objective preparations. When preparing compositions for injection, a pharmacologically effective amount of the human-type antihuman IgE monoclonal antibody of the present invention may be mixed with pharmaceutically acceptable vehicles and/or activation agents, such as amino acids, saccharides, cellulose derivatives, and other organic and/or inorganic compounds. In addition, when preparing a composition for injection from the antibody of the present invention and such vehicles and/or activation agents, a pH adjusting agent, buffering agent, stabilizer, solubilizing agent, and the like, may be added according to a conventional method as required.

The antibody of the present invention can also be used as an immunotoxin. Immunotoxins were initially prepared as chimeric molecules by chemically combining an antibody molecule and a toxic substance (Vietta et al., Cell, 41, 653-654 (1985); Pastan et al., Ann. Rev. Biochem. 61, 331-354 (1992)). In these molecules, the antibody portions contribute to selective binding to the target cells, whereas the toxic portions contribute to the introduction to cytoplasm, thereby contributing to death of the cells thereafter. Several types of toxins have been used for the preparation of immunotoxins. For example, ricin A chain, blocked ricin, saporin, pokeweed antiviral protein, diphtheriatoxin and Pseudomonas exotoxin A (Pastan et al., Science 254, 1173-77 (1991); Vietta et al., Semin. Cell Biol. 2, 47-58 (1991); Tazzari et al., Br. J. Hematol. 81, 203-211 (1992); Uckun et al., Blood, 79, 2201-14 (1992)), and the like are known. These toxins are proteins resulting from plants or bacteria and generally consist of A chains and B chains, which are bonded by disulfide bonds. The A chain has the activity of inhibiting protein synthesis and killing cells if introduced into cytoplasm. The B chain mediates introduction of the A chain into cells. An immunotoxin can be prepared using only an A chain that exhibits fewer side effects than a complete toxin or using a combination of the A chain and an A chain mutant, and producing as a molecule in which the A chain or the A chain/A chain mutant is combined with the antibody or part of the antibody. Either a chemical reaction for causing the toxin and antibody to bond or a gene recombination technique by which the immunotoxin is prepared as a fusion protein using *Escherichia coli* or the like as a host microorganism can be used for the preparation of the immunotoxin. Since lymphocytes producing an IgE cause allergic reactions, the immunotoxin made from the antibody of the present invention or a fragment thereof and a toxic substance can remove IgE-producing lymphocytes, whereby occurrence of an allergy is prevented or allergic diseases can be treated.

### BRIEF DESCRIPTION OF THE DRAWINGS

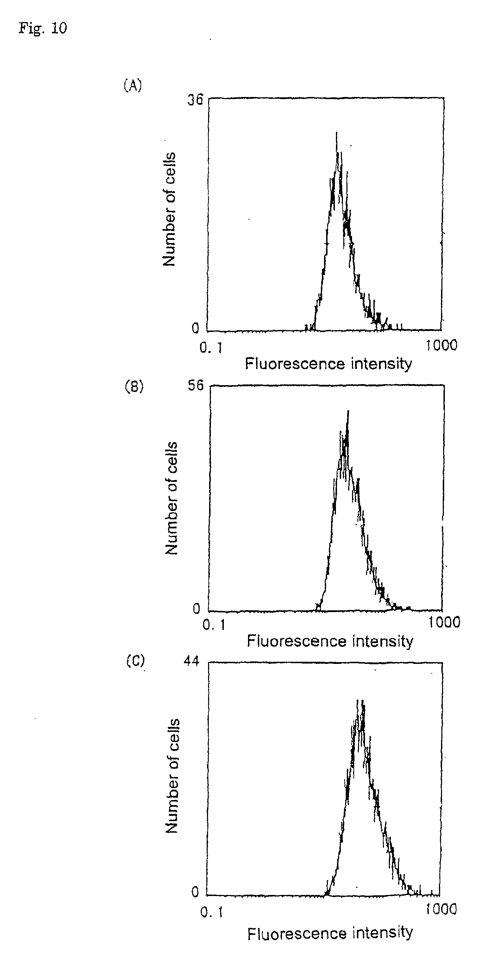

Figure 1 shows the results of EIA analysis of the binding of a purified human-type antihuman IgE monoclonal antibody to P18 peptide.
   □: Antibody 1012G of the present invention
   ◆: Antibody 72D of the present invention
   ○: Antibody 611B of the present invention
   ▲: Antibody 46E of the present invention
Figure 2 shows a map of antibody H-chain expression vector pCISRα/H.
Figure 3 shows a map of antibody L-chain expression vector pCISRα/L.
Figure 4 shows a map of antibody H+L-chain expression plasmid pCISRα/HL.
Figure 5 shows a map of plasmid pUCDHFR used for gene amplification using MTX.
Figure 6 shows the results of *in vitro* analysis of the immune complex forming capability of the antibody of the present invention by the EIA method.
   □: Antibody 72D of the present invention
   ○: Mouse chimera antibody (M#9Ch)
Figure 7 shows the results of *in vitro* analysis of the immune complex forming capability of the antibody of the present invention by the EIA method when IgE is directly immobilized.
   □: Antibody 72D of the present invention
   ○: Mouse chimera antibody (M#9Ch)
Figure 8 shows the results of *in vitro* analysis of the binding activity of the antibody of the present invention to IgE-producing cells by the EIA method.
Figure 9 shows the results on the effect of the antibody of the present invention to decrease an *in vivo* free IgE concentration over time, wherein

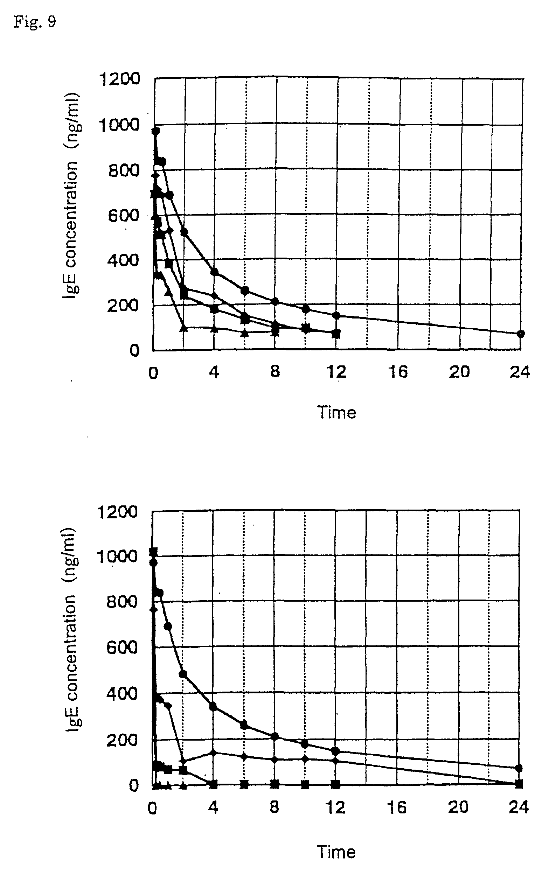

   Figure 9 (1) is a result using a solvent and mouse chimera antibody, wherein
   ◆: Mouse chimera antibody (M#9Ch), dose: 10 µg/kg
   ■: Mouse chimera antibody (M#9Ch), dose: 100 µg/kg
   ▲: Mouse chimera antibody (M#9Ch), dose: 1,000 µg/kg
   ●: Solvent
   Figure 9 (2) is a result using a solvent and the antibody of the present invention, wherein
   ◆: Antibody 72D of the present invention, dose: 100 µg/kg
   ■: Antibody 72D of the present invention, dose: 300 µg/kg
   ▲: Antibody 72D of the present invention, dose : 1,000 µg/kg
   ●: Solvent
Figure 10 shows the reactivity of the antibody of the present invention to IgE-treated basophiles, wherein
Figure 10 (A) is the result obtained when the added amount of the antibody of the present invention is 0 µg/ml,
Figure 10 (B) is the result obtained when the added amount of the antibody of the present invention is 1 µg/ml, and
Figure 10 (C) is the result obtained when the added amount of the antibody of the present invention is 10 µg/ml.

### THE PREFERRED EMBODIMENT OF THE INVENTION

### Examples

The present invention will now be described in more detail by way of examples , which are given for the purpose of explanation and should not be construed as limiting the present invention.

### Example 1

### <Establishment of hybridoma cells producing human-type antihuman IgE monoclonal antibody>

### (1) In vitro antigen sensitization

Peripheral lymphocytes obtained from two healthy persons with different allotypes were melted at 37°C and washed twice by centrifugation (for 10 minutes at 250 x g) using RPMI-1640 culture medium (Gibco BRL Co.) . The same number of lymphocytes were mixed and suspended in a culture medium containing 2.5 mM leucine-o-methyl ester (Aldrich Co.). The suspension was allowed to stand for 40 minutes at room temperature. The cells were washed with a RPMI-1640 culture medium and suspended in the RPMI-1640 culture medium to a concentration of 10⁷ cells/ml. Muramyl dipeptide (40 µg/ml; Sigma company) , human IL-4 (100 U/ml; Peprotech Co.), and Human IL-6 (10 ng/ml; R&D Systems, Inc.) were added to the resulting suspension, which was charged to a 6-well plate, 2.5 ml per well. Various amounts (0.1-10 µg) of sensitized antigen (human IgE; The Scripps Research Institute) were added and the mixtures were allowed to stand for 15 minutes at room temperature. Then, a RPMI-1640 culture medium containing 40% fetal calf serum (FCS) was added in the amount of 2.5 ml to each mixture, followed by incubation for 3-5 days in 5% CO₂ at 37°C. 25 mM HEPES, 100 U/ml penicillin, 100 µg/ml streptomycin, 2 mM sodium pyruvate, 2 mM non-essential amino acids, and 5x10⁻⁵ M 2-mercaptoethanol were added to the RPMI-1640 culture medium containing 10% FCS. The resulting medium was used as a cell culture medium (CRPMI). The collected lymphocytes were centrifuged for 5 minutes at 250 x g, washed twice, and suspended in the cell culture medium.

### (2) Cell fusion

K6H6/B5 heteromyeloma cells (ATCCCRL1832) in a logarithmic growth phase were collected by centrifugation for 5 minutes at 250 x g. The cells were washed with a RPMI-1640 culture medium and the number of cells was counted. Sensitized lymphocytes and K6H6/B5 cells at a ratio of 2:1 to 4:1 were charged into a 50 ml conical tube and mixed. The mixture was centrifuged for 5 minutes at 250 x g to completely remove the culture medium. The resulting cells were gently shaken to disentangle the pellets. 1ml of RPMI1640 solution containing 42% (w/v) polyethylene glycol 3350 (Merck Co.) and 17% (v/v) dimethylsulfoxide was slowly added over one minute while rotating the tube. 10 ml of serum-free RPMI-1640 culture medium was slowly added while gently stirring. The cells were centrifuged for 5 minutes at 250 x g to remove the culture medium. Then, K6H6/B5 cells were suspended in a HAzT culture medium (CRPMI medium containing hypoxanthine (0.1 mM), Azaserine (1 µg/ml, Sigma Corp.), thymidine (16 µM), and Human IL-6 (1 ng/ml)) to a concentration of 2.5 x 10⁵ cells/ml. Fused cells were inoculated to a 96-well micro-plate, 0.2 ml/well, and cultured in a 5% CO₂ incubator at 37°C. After 4 days, 50 µl/well of fresh HAzT medium was added. Thereafter, 50 µl of culture medium in each well was replaced with fresh medium every five days.

### Example 2

### <Selecting and cloning of hybridoma producing human-type antihuman IgE monoclonal antibody>

The antigen specificity of the antibody produced by the hybridoma was examined by the ELIZA method for each well in which cell growth was recognized. Human IgE (2 µg/ml) dissolved in 0.1 M NaHCO₃ (pH 9.6) was added in an amount of 100 µl per well, and the antigen was immobilized on a membrane (Biodyne A; German Science Co.) by suction. Next, 200 µl of 2% bovine serum albumin (BSA)/10 mM phosphate buffer solution (pH 7.4) in 0.15 M NaCl (PBS) was added to each well to block remaining binding sites. After the addition of 100 µl of a supernatant of cultured medium, the mixture was reacted for 2 hours at room temperature. After washing with PBS-0.05% Tween 20 (PBST) four times by suction, 100 µl/well of alkali phosphatase (AP)-labeled goat antihuman IgG antibody (Capel Corp.) diluted to 10,000 fold with 0.5% BSA/PBS was added in order to detect the bound antibody, followed by reaction for two hours at room temperature. After washing with PBST four times by suction, 100 µl of an AP substrate solution was added to cause the reactant to color at room temperature. The reactant was then transferred to a 96 well micro-plate to measure absorbance at 405 nm using a micro-plate reader (Immuno Reader NJ-2000; Nalge Nunc International). Cells producing an antigen specific antibody were mono-cloned by performing cloning three times using a limiting dilution method (0.3/well).

### (1) Synthesis and purification of P18

P18 (a peptide of the present invention designed from 18 amino acids derived from CH2-CH3 region of human IgE) was synthesized from 0.25 mmol HMP resin (Applied Biosystems Inc.) and 1 mmol Fmoc amino acid (Applied Biosystems Inc.) by the Fmoc method using a peptide synthesizer (Model 431A; AppliedBiosystems Inc.). 10 ml of a removing solution (Applied Biosystems Inc.) was added to the resulting peptide resin (1,047 mg) . The mixture was reacted for 4 hours at 4°C and 1.5 hours at room temperature to remove the peptide from the resin. The solution obtained by filtration through a glass filter was concentrated. Then, cold diethyl ether was added to cause the peptide to precipitate. The precipitated peptide was collected by a glass filter. The peptide was dissolved in 2N acetic acid solution and lyophilized to obtain 392 mg of a crude peptide. 150 mg of the crude peptide was purified by reverse phase HPLC (capsule pack C18; Shiseido Co., Ltd.) to obtain P18 (94 mg) with a purity of 98%.

### (2) Binding activity of the antibody to P18

The human-type antihuman IgE monoclonal antibody was selected from human-type antihuman IgE monoclonal antibodies produced by the established hybridoma cells by the EIA method using the binding activity with the peptide of the present invention (P18; Sequence ID No. 1 in the Sequence Table) designed from 18 amino acids originating from a CH2-CH3 region of human IgE as an indicator. A solution of sodium p-nitrophenyl phosphorate (Sigma 104; Sigma Co.) in 1 M diethanolamine/0.5 mM magnesium chloride (pH 9.8) (concentration: 1 mg/ml) was used as an AP substrate solution. After washing a 96-well micro-plate, which had been coated with 2% BSA overnight at 4°C, with PBST, 100 µl of a P18 / glutaraldehyde (0.25%; Sigma Co.) / PBS (10 mg/ml) solution was added to each well, followed by a reaction for one hour at room temperature. After washing with PBST, a 40 mM Tris-HCl buffer (pH 7.4) containing 0.02% NaN₃ was added to block overnight at 4°C, thereby obtaining a P18 immobilized plate. After the addition of 100 µl of a cell culture supernatant of cultured medium, the mixture was reacted for 2 hours at room temperature. After washing three times with PBS, 100 µl of AP-labeled antihuman IgG antibody (Capel Corp.) diluted to 1,000 fold with 1% BSA/PBS was added, followed by reaction for two hours at room temperature. After washing, 100 µl of an AP substrate solution was added to cause the reactant to color at room temperature. The absorbance at 405 nm was measured using a micro-plate reader. Four antibodies (1012G, 72D, 611B, and 46E) exhibiting binding activity with P18 were selected from about 2000 wells (estimated 200,000 clones) by this method. The results are shown in Table 1.

**Table 1**

| Antibody | Absorbance |
|---|---|
| 1012G | 0.601 |
| 611B | 0.073 |
| 46E | 0.046 |
| 72D | 0.045 |

### Example 3

### <Preparation and purification of antibody of the present invention>

Hybridomas producing four antibodies grown in a RPMI-1640 culture medium containing 5% FCS were washed three times with PBS, respectively, and suspended in a serum-free RPMI-1640 culture medium to a concentration of 10⁶ cells/ml, and incubated for 3 days in 5% CO₂ incubator at 37°C. Ammonium sulfate (Nacalai Tesque Co.) was added to the supernatant of cultured medium centrifugally obtained to a final concentration of 40%, the pH was adjusted to neutral, and the mixture was allowed to stand for one hour at 4°C to effect salting-out. Precipitate obtained by centrifugation (7000 x g for 20 minutes) was dissolved in a small amount of PBS and dialyzed against PBS at 4°C to remove ammonium sulfate. Finally, after dialyzing with a 20 mM phosphate buffer solution (pH 8.0), insoluble matters were removed by centrifugation, and the sample was filtered. NaN₃ was then added to a final concentration of 0.02%. The sample was applied to Protein G-Sepharose 4B column (Zymed Laboratories, Inc.; 2 ml) equilibrated with a 20 mM phosphate buffer solution (pH 8.0) and washed with 30 ml of equilibrated buffer solution. The antibody was eluted with 8 ml of ice-cold 0.1 M glycine HCl buffer solution (pH 3.0) and immediately neutralized with 0.4 ml of 1.5M Tris-HCl buffer solution (pH 8.9). After the antibody solution was dialyzed with PBS at 4°C, the absorbance at 280 nm was measured to calculate the antibody concentration (E1% 13.5). The results are shown in Table 2. The isotype of the resulting antibodies was examined by the ELISA method to confirm that all four antibodies were classified as IgG1 (λ).

**Table 2**

| Antibody | Amount of serum-free culture (ml) | Amount of purified antibody (mg) |
|---|---|---|
| 1012G | 400 | 2.93 |
| 611B | 450 | 2.14 |
| 46E | 450 | 5.63 |
| 72D | 300 | 2.26 |

### Example 4

### <Antigen recognition by the antibody of the present invention>

### (1) Affinity to P18

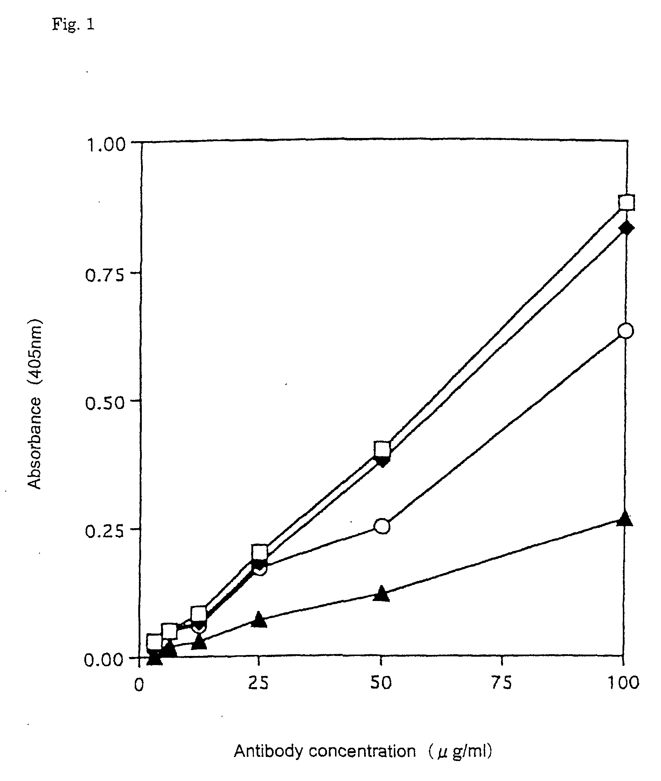

Affinity to P18 peptide of the four purified human-type antihuman IgE monoclonal antibodies diluted with 1% BSA/PBS to various concentrations was investigated in the same manner as in Example 2. The results are shown in Figure 1. As a result, all four antibodies have been confirmed to possess affinity to P18.

### (2) Binding inhibiting activity of human IgE

Inhibiting activity of human IgE forwards the binding of the antibodies of the present invention to P18 was investigated using the competitive inhibition method. 0.2 mg of antibody was mixed with various amounts of human IgE and reacted for one hour at 37°C. The reactant was added to a micro-plate in which P18 was immobilized and incubated for two hours at room temperature. After washing, 100 µl of AP-labeled antihuman IgG antibody (Capel Corp.) diluted to 1,000 fold with 1% BSA/PBS was added, followed by reaction for two hours at room temperature. After washing, 100 µl of an AP substrate solution was added to cause the reactant to color at room temperature. Then, the absorbance at 405 nm was measured. The results are shown in Table 3. As a result, all four antibodies have been confirmed to combine with IgE due to inhibition of bonding to P18 by pretreatment with the human IgE.

**Table 3**

| Added human IgE (µg/ ml) | Binding inhibition (%) | | | |
|---|---|---|---|---|
| | 46E | 611B | 72D | 1012G |
| 0 | 0 | 0 | 0 | 0 |
| 22.5 | 46 | 32 | 17 | 44 |
| 45 | 66 | 33 | 51 | 65 |
| 90 | 76 | 72 | 74 | 85 |

### (3) Binding activity of antibody of the present invention to human IgE-producing cells

Human IgE-producing myeloma cells (SKO-007; ATCC CRL-803 3-1) were suspended in PBS-0.02 % NaN₃ to a concentration of 2.5x10⁶ cells/ml. After the same amount of antibodies of the present invention dissolved in 2% BSA/PBS (20 µg/ml) was mixed, the mixture was reacted for one hour at 37°C. After washing, cells were suspended in AP-labeled antihuman IgG antibody (Capel Corp.) diluted to 1,000 fold and reacted for two hours at room temperature. After washing with PBST, 0.5 ml of an AP substrate solution (sodium p-nitrophenyl phosphate (2 mg/ml)) was added to each sample. The supernatant liquid was sampled to measure the absorbance at 405 nm. The results are shown in Table 4. As a result, all four antibodies of the present invention have been confirmed to bind to human IgE-producing cells. In addition,these antibodies have been confirmed not to combine with IgG-producing cells IM-9 (ATCC CCL-159) and IgM-producing cells RPMI-1788 (ATCC CCL-156), according to the same method.

**Table 4**

| Antibody | Absorbance (405 nm) | |
|---|---|---|
| | Experiment 1 | Experiment 2 |
| Normal IgE | 0.060 | 0.037 |
| 1012G | ND | 0.795 |
| 611B | 0.861 | 1.090 |
| 46E | 0.186 | ND |
| 72D | 0.626 | ND |
| ND indicates the experiment was not carried out. | | |

### Example 5

### <Histamine liberation from peripheral mononuclear cells of the antibodies of the present invention>

Mononuclear cells separated from human peripheral blood using Ficoll-Conray (Lymphosepar I; Immuno-Biological Laboratories. Co., Ltd.) were sensitized with IgE. After the addition of the antibodies (0.1 µg) of the present invention, the mixture was reacted for 30 minutes at 37°C. Histamine liberated from the mononuclear cells was determined using an HRT kit (Miles Corp.) in accordance with the attached protocol. The results are shown in Table 5. As a result, the amount of histamine liberated from the peripheral mononuclear cells which have been treated by the antibody 72D among the antibodies of the present invention was least.

**Table 5**

| Antibody | Released histamine concentration (nM) |
|---|---|
| 1012G | 2.09 |
| 611B | 3.11 |
| 46E | 4.51 |
| 72D | <1.5 |

### Example 6

### <Cloning of antibody cDNA>

### (1) Preparation of cDNA library

32.8 µg of poly (A) +RNA was obtained from 10⁸ hybridoma cells (72D) using an mRNA separation kit (Invitrogen Inc.). Using 5 µg of this mRNA as a template, a double strand cDNAwas synthesized using a cDNA synthesis kit (Amarsham Co.) . A half amount of the cDNA (1.86 µg) thus obtained was subjected to the addition of *EcoR* I-*Not* I-*BamH* I adapter (Takara Shuzo Co., Ltd.) to both terminals, followed by gel chromatography using sepharose as a carrier, thereby separating fractions containing cDNA. A cDNA library was prepared by incorporating the cDNA into an *EcoR* I arm of the phage vector λgt10 according to the method of Huynh, T. V. et al. (DNA cloning; a practical approach, p48-78, edited by Glover, D. M. (1985), published by IRL Press).

### (2) Screening of cDNA library

The recombinant phage DNA obtained above was in vitro packaged using Gigapack Gold (Stratagene Co.) to cause the DNA to be infected with *Escherichia coli*, thereby obtaining 1.7x10⁴ phage plaques. The plaques were adsorbed in a Hybond-N filter (Amarsham Co.) to obtain a replica filter. Then, plaques containing either H-chain cDNA or L-chain cDNA of the antibody were screened respectively by a plaque hybridization method using a DNA fragment encoding a constant region of the antibody gene labeled with ³²P as a probe. As a result, several tens of positive plaques were recognized for both the H-chain and the L-chain. These positive plaques were independently collected in 500 µl of an SM buffer and were stored as phage stocks. Phage DNAs were collected from the stocks, digested with restriction endonuclease *Not* I, and subjected to agarose gel electrophoresis to confirm the length of DNA fragments. DNA fragments judged to contain the entire length of cDNA were purified. H-chain cDNA with a length of about 1.7 kb and L-chain cDNA with a length of about 0.9 kb were obtained from selected phage stocks H53 and L5, respectively.

### (3) Determination of cDNA base sequence

The full length of cDNA thus obtained was inserted into a plasmid vector pBLUE SCRIPT II SK+ (Toyobo Co., Ltd.), which had previously been digested, using a ligation kit (Takara Shuzo Co., Ltd.) to perform transformation of *Escherichia coli*. As a result, a subclone containing one copy of the H-chain cDNA and L-chain cDNA was obtained. A recombinant plasmid was collected from the subclone and used as a sequencing template. A DNA Sequencing Kit (Perkin Elmer Co.) was used to carry out the reaction according to the attached protocol for sequencing. The products were analyzed using an automatic DNA sequencer (PRISM model 377; ABI Co.) to determine the whole base sequence. The determined sequences for nucleic acid are shown in the Sequences ID No. 2 (H-chain) and ID No. 3 (L-chain) and the amino acid sequences translated from the nucleotide sequences are shown in the Sequences ID No. 10 (H-chain) and ID No. 11 (L-chain) of the Sequence Table, respectively.

The entire nucleotide sequences were determined in the same manner for the three other antibodies (1012G, 611B, and 46E) obtained. The nucleotide sequences determined for the antibody 1012G are shown in the Sequences ID No. 4 (H-chain) and ID No. 5 (L-chain) and the amino acid sequences translated from the nucleotide sequences are shown in the Sequences ID No. 12 (H-chain) and ID No. 13 (L-chain) of the Sequence Table. The nucleotide sequences determined for the antibody 611B are shown in the Sequences ID No. 6 (H-chain) and ID No. 7 (L-chain) and the amino acid sequences translated from the nucleotide sequences are shown in the Sequences ID No. 14 (H-chain) and ID No. 15 (L-chain) of the Sequence Table. The nucleotide sequences determined for the antibody 46E are shown in the Sequences ID No. 8 (H-chain) and ID No. 9 (L-chain) and the amino acid sequences translated from the nucleotide sequences are shown in the Sequences ID No. 16 (H-chain) and ID No. 17 (L-chain) of the Sequence Table.

### Example 7

### (1) Construction of vector for expression of antibody

An expression vector was prepared by replacing the CMV/IE enhancer/promoter region of a mammal expression vector (pCI; Promega Co.) with an SRα promoter region containing 16S splice junction of expression vector pcDL-SRα296 (Takebe, Y., et al., Mol. and Cell. Biol., (1988) 466-472). The antibody expression vector was constructed based on this expression vector. Specifically, a mammal expression vector pCI was digested with *Bgl* II and the terminals were blunted using a DNA Blunting kit (Takara Shuzo Co., Ltd.). The vector was purified by extraction with phenol/chloroform and precipitation in ethanol. The purified vector was digested with *Pst* I and the vector not containing a CMV/IE enhancer/promoter region was purified using gel. The expression vector pcDL-SRα296 was digested with *Hind* III, blunted in the same manner as above, and digested with *Pst* I. The resulting product was subjected to agarose gel electrophoresis to purify an SRα promoter region DNA containing 16S splicing j unction . This DNA and the pCI vector not containing a CMV/IE enhancer/promoter region were ligated using a DNA Ligation kit (Takara Shuzo Co., Ltd.) to obtain a vector (pCISR α) for expression of the antibody H-chain.

In addition, a vector (pCICla) obtained by introducing ClaI phosphorylation connecting unit (Nippon Gene Co.) into the *Bgl* II site of the expression vector pCI was digested with *Bal* I and the terminals were blunted, followed by purification by extraction with phenol/chloroform and precipitation in ethanol. The purified vector was digested with *Pst* I and a vector free of a CMV/IE enhancer/promoter region was purified using gel. This DNA and the SR α promoter region DNA containing 16S splicing junction were ligated in the same manner as above to obtain a vector (pCIClaSR α) for expression of the antibody L-chain.

### (2) Construction of antibody H-chain expression vector

A DNA fragment (SigVHCH 1) containing part of a signal sequence, VH and CH1 was obtained by the PCR method (25 cycles; each 1 minute at 94°C, 55°C, and 72°C) using the antibody H-chain cDNA prepared from the hybridoma (72D) for producing the antibody of the present invention as a template and *Sal* I sigVH1F (Sequence ID No. 18) and HFR4 CH1A paIR (Sequence ID No. 19) as primers. In addition, a CH fragment was obtained by the PCR method using ApaICH1F (Sequence ID No. 20) and CH4Eco52IR (Sequence ID No. 21) as primers.

### PCR conditions

| | |
|---|---|
| Takara Shuzo Co., Ltd. ExTaq (5 U/µl) | 0.5 µl |
| 10x ExTaq buffer | 10 µl |
| dNTP Mixture (2.5 mM for each) | 8 µl |
| Template | About 100 µg |
| Primer (2-types) | for each 0.2 µM |
| Make 100 µl with the addition of sterile distilled water. | |

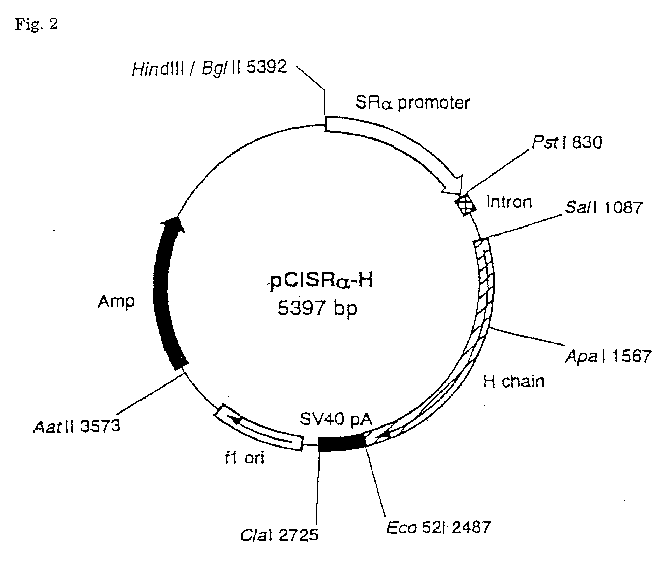

The PCR products obtained were cloned using a pT7Blue T-vector kit (Novaken Co., Ltd.). Specifically, T4 DNA ligase (4U) was added to a ligase reaction buffer solution containing about 20 ng of each PCR product purified by gel filtration and 50 ng of pT7BlueT vector digested with restriction endonucleases (*Apa* I and *Eco* 52I), and the mixture was maintained at 16°C for two hours. Next, 2 µl of the above ligase reaction solution was mixed with 50 µl of a competent *Escherichia coli* (DH5 α) to which 2 µl of 0.5 M 2-mercaptoethanol was added. The mixture was allowed to stand for 30 minutes on ice, heated for 40 seconds at 42°C, and again allowed to stand for 2 minutes on ice. Then, 0.5 ml of SOC culture medium was added and the mixture was shake-cultured at 37°C for 1 hour. This culture broth was spread over an LB plate containing 0.1 mg/ml ampicillin and incubated overnight at 37°C. Single colonies produced in the plate were picked up and cultured in an LB culture medium (LB-Amp, 1ml) containing 0.1 mg/ml ampicillin overnight at 37°C. Cells were collected from the culture broth, and a plasmid DNA was prepared in accordance with an alkaline method. Transformed *Escherichia coli* possessing a plasmid having a proper nucleotide sequence was obtained as a result of DNA sequence analysis. The plasmid DNA in the culture broth obtained by culture overnight (5 ml) was purified and a vector containing SigVHCH1 was digested with restriction endonucleases (*Apa* I and *Eco* 52I). The vector was purified by extraction with phenol/chloroform and precipitation in ethanol. The vector containing the CH fragment was digested with restriction endonucleases (*Apa* I and *Eco* 52I) and purified by gel filtration to obtain restriction endonuclease-treated fragments. 8 µl (about 20 ng) of the CH fragment was added to 1 µl (about 50 ng) of an enzyme-treated vector. 10 µl of DNA ligation kit Ver. 2 I solution (Takara Shuzo Co., Ltd.) was added to the mixture, followed by a ligation reaction for two hours at 16°C. The reaction product was added to a competent TG1 (500 µl), and the mixture was allowed to stand for 45 minutes at 0°C and transformed by a heat shock (at 42°C for 2 minutes). 100 µl of the resulting product was suspended in 900 µl of LB/20 mM glucose (LBG) culture medium and the mixture was shake-cultured at 37°C for 1 hour. The transformed cell suspension diluted with LBG at various concentrations was spread over a SOBAG plate, and incubated overnight at 37°C. Single colonies produced were picked up, suspended in 1 ml of LB-Amp culture medium, and incubated for 16 hours at 37°C. Cells were collected from the culture broth and plasmid DNA was prepared by an alkaline method to analyze the nucleotide sequence. As a result, a transformed *Escherichia coli* possessing a plasmid (pT7 Blue/H-72D) having a proper antibody cDNA nucleotide sequence was obtained. The plasmid DNA in the culture broth obtained by culture overnight was purified, and digested with restriction endonucleases (*Sal* I and *Eco* 52I) and purified. The purified substance was ligated with the above plasmid vector (pCISRα) for H-chain expression digested with restriction endonucleases (*Sal* I and *Eco* 52I) to obtain a vector (pCISRα/H) for expression of the antibody H-chain. Figure 2 shows a map of an antibody H-chain expression vector.

### (3) Construction of antibody L-chain expression vector

A DNA fragment (SigVLCL) containing a signal sequence, VL and CL, was obtained by the PCR method using the antibody L-chain cDNA prepared from the hybridoma (72D) for producing the antibody of the present invention as a template and *Sal* I sigVL1F (Sequence ID No. 22) and CLNotIR (Sequence ID No. 23) as primers. The PCR product obtained was cloned with a pT7Blue T-vector in the same manner as above to transform the *Escherichia coli*. *Escherichia coli* holding a plasmid (pT7Blue/L-72D) introduced an antibody L-chain indicating a proper nucleotide sequence was obtained.

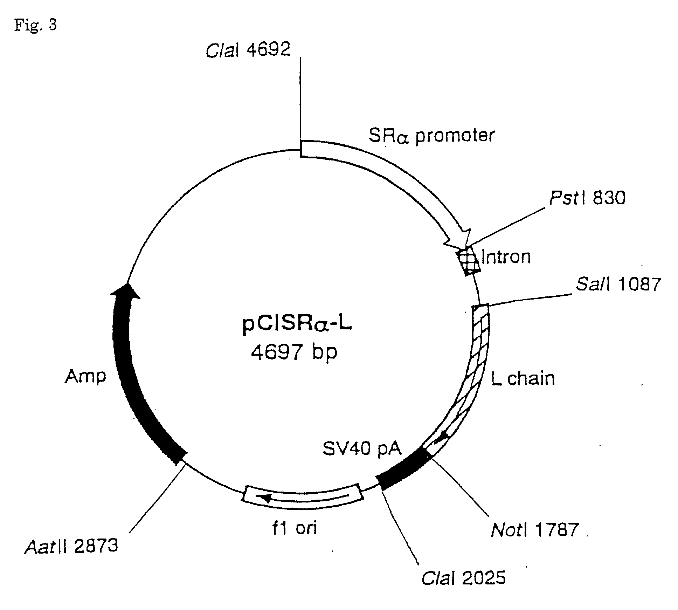

The antibody L-chain DNA was purified by purifying the plasmid DNA in the culture broth obtained by culture overnight and digesting the plasmid DNA with restriction endonucleases (*Sal* I and *Not* I) . A vector (pCIClaSR α) for the expression of antibody L-chain digested with restriction endonucleases in the same manner and the antibody L-chain DNA were respectively ligated to prepare an antibody L-chain expression vector (pCISRα/L). A map of an antibody L-chain expression vector is shown in Figure 3.

### (4) Construction of antibody (H+L) expression vector

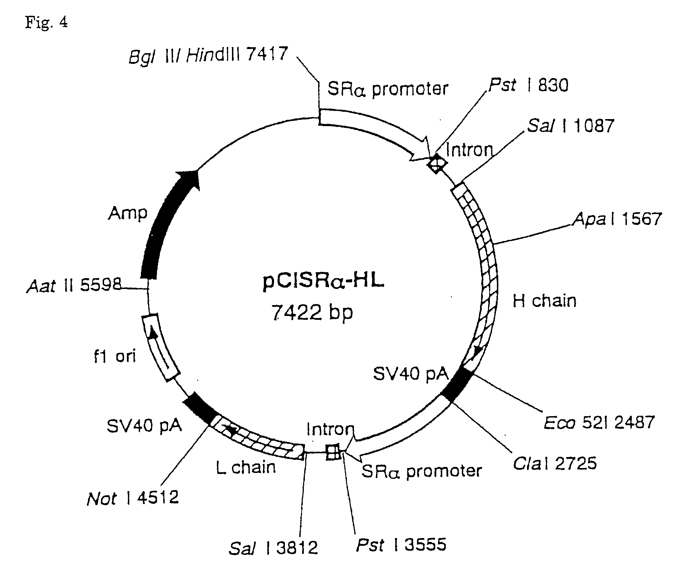

Antibody L-chain expression plasmid (pCIClaSR α/L) was digested with restriction endonucleases (*Aat* II and *Sal* I). The fragments containing an antibody L-chain DNA (*Sal* I/*Aat* II) were purified by gel filtration. The remaining vector fragments were further digested with *Cla* I and a DNA fragment (*Cla* I/*Sal* I) containing a promoter region was purified in the same manner. Antibody H-chain expression plasmid (pCISR α/H) was digested with restriction endonucleases (*Aat* II and *Cla* I). The vector fragments containing an antibody H-chain DNA (*Cla* I/*Aat* II) were purified by gel filtration. *Escherichia coli* (DH5α) was transformed by ligation of the above vector fragments with corresponding *Sal* I/*Aat* II fragments and *Cla* I/*Sal* I fragments originating from the antibody L-chain expression plasmid, thereby obtaining *Escherichia coli* holding antibody H+L-chain expression plasmid (pCISRα-HL; Figure 4). The *Escherichia coli* was named DH5a/pWT-Z and deposited with the Biotechnology Laboratory, National Institute of Advanced Science and Technology, the Ministry of Economy, Trade and Industry (1-3, Higashi 1-Chome, Tsukuba-shi, Ibaraki-ken, Japan 305-3566) on January 27, 1999 (FERM BP-7344, transferred from P-17173 on January 27, 1999).

### Example 15

### <Plasmid large-scale preparation and electroporation>

A glycerol stock of *Escherichia coli* transformed with an antibody expression vector was streaked onto an ampicillin-containing LB plate. Single colonies produced were inoculated into 20 ml of an ampicillin-containing terrific broth (TB-Amp) and shake-cultured overnight at 37°C. Four 2 1 conical flasks with a baffle, each containing 400 ml of TB-Amp, were charged with 5 ml of *Escherichia coli* culture broth. The *Escherichia coli* was cultured at 37°C while rotating at 160 rpm. When the absorbance at 600 nm reached 0.8-1.0, spectinomycin (Sigma Co.; final concentration 0.17 mg/ml) was added, and culture of the microorganisms was continued for about 20 hours. *Escherichia coli* was collected from the culture broth by centrifugation (4,000xg, for 10 minutes at 4°C) and 9.0 mg of plasmid DNA was purified using a Plasmid Mega Kit (Qiagen Company).

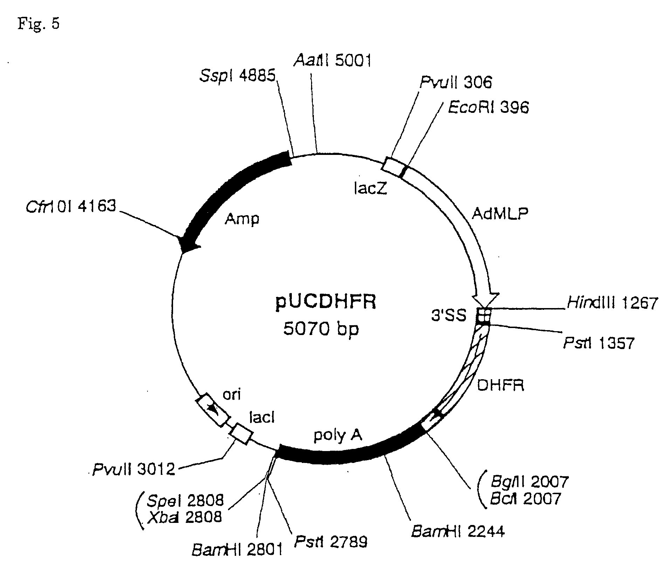

The plasmid DNA obtained in a final amount of 3 ml was digested with restriction endonuclease *Aat* II (2000U) overnight at 37°C. The digested plasmid DNA was subjected to extraction with phenol, extraction with chloroform, and ethanol precipitation. The precipitate was re-dissolved in TE. After mixing 200 µg of the antibody expression vector and pUCDHFR (20 µg; Figure 5) digested with Aat II, the mixture was again aseptically subjected to precipitation using ethanol. The precipitate was dissolved in 20 µl of TE and 80 µl of IMDM culture medium (IMDM-10) containing 10% FCS was added.

2x10⁷ floating CHO cells (dhfr-) were suspended in 700 µl of IMDM-10. After the addition of the above DNA solution, the mixture was transferred to a cuvette (Gene pulser cuvette; 0.4 cm electrode; Biorad Co.). A gene was introduced by electroporation under the conditions of 330 V and 960 µF. After standing still for 10 minutes, the DNA was suspended in PF325 culture medium (JRH Bioscience Corp.) added HT (Gibco BRL Co.), followed by culturing for 2 days at 37°C. Cells were suspended in the PF325 culture medium to a concentration of 10⁴ cells/ml and inoculated to a 96-well micro-plate (Nunc Co.; MaxiSorp), at 200 µl/well, and cultured in a 5% CO₂ incubator at 37°C.

### Example 16

### <Detection of antibody expression cells>

100 µl of a supernatant of cultured medium was added to a 96 well micro-plate, on which 1 µg of antihuman IgG (Fc) antibody (Capel Corp.) had been immobilized, allowed to stand for two hours at room temperature, and washed 6 times with PBS T. 25% Block Ace (BA; Snow Brand Milk Products Co., Ltd.) and AP labeled antihuman λ-chain antibody (Bio Source Co.; 2,000 fold dilution) dissolved in PBS were added, each in the amount of 100 µl, and the mixture was allowed to stand for two hours at room temperature. After washing, an AP substrate solution was added in the amount of 100 µl for each and the mixture was reacted for at room temperature. After terminating the reaction with a 3N-NaOH solution, the absorbance at 405 nm was measured using a micro-plate reader (NJ-2000; Japan Intermed Co. , Ltd.). As a result, cells in several wells exhibiting high absorbance were selected.

### Example 17

### <Gene amplification and cloning>

After amplification of selected antibody expression cells, the cells were suspended in the PF325 culture medium containing 20 nM methotrexate (Mtx; Sigma Co.) and seeded to a 96-well micro-plate, 10⁴ cells/0.2 ml/well, and cultured in a CO₂ incubator at 37°C. MTX resistant cells were selected, inoculated in a 12 well plate at a concentration of 25x10⁴ cells/ml, and cultured for four days. The antibody concentration of the supernatant of cultured medium was measured to select cells exhibiting high productivity. A purified human IgG (λ) (Nordic Immunology Co., Ltd.) was used as a standard sample. The cells were seeded in a 96-well micro-plate at a concentration of 100 cells/well and cloned to select cells exhibiting high productivity in a same manner. Gene amplification and cloning were repeated using culture media in which the MTX concentration was stagedly increased, thereby establishing a high productivity cell strain.

### Example 18

### <Production and purification of recombinant antibody>

The high productive cell strain obtained above was suspended in ExCell 301 culture medium containing MTX (Nichirei Corp.) to a concentration of 25x10⁴ cells/ml and incubated for 3 days at 37°C in a CO₂ incubator. The cells were aseptically separated from the culture broth. The collected cells were suspended in a fresh culture medium to a concentration of 25x10⁴ cells/ml and cultured for three more days. This procedure was repeated to obtain 92.3 l of the culture broth containing the antibody. The culture broth containing the antibody was adjusted to pH 7.4 with 6N-NaOH and filtered (millipack 0.22 µm; Millipore Co.).

The filtered antibody solution was applied to HiTrap rProtein A (5ml; Pharmacia Biotech Inc.) equilibrated with PBS. After washing the column with PBS, the antibody was eluted using a 0.1 M sodium citrate buffer solution (pH 3.0). The eluted material was immediately neutralized with a 1.5M Tris-HCl buffer solution (pH 8.7), thereby obtaining a purified recombinant antibody. As a result, 1.70 mg of purified antibody was obtained from the culture broth containing 1.86 mg of the antibody 72D.

### Example 19

### <Evaluation of the antibody of the present invention by in vitro experiment>

### (1) Immune complex forming capability

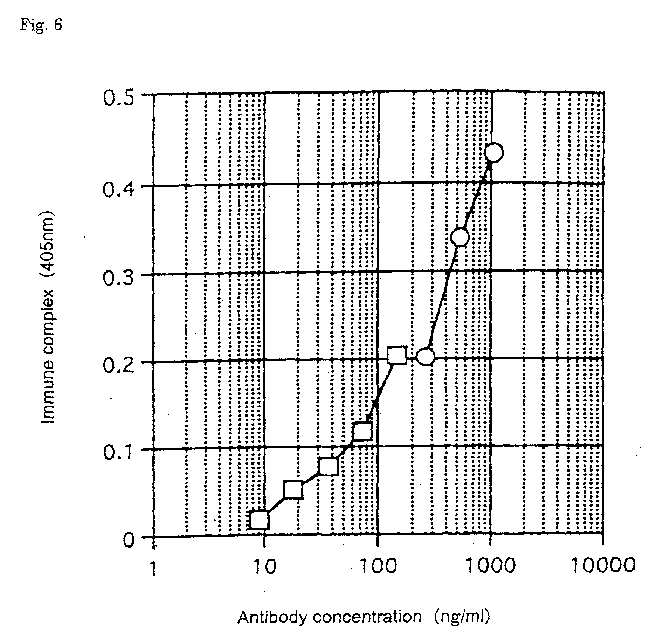

Human IgG (Scripps Research Institute; final concentration 0.5 µg/ml) and various amounts of antihuman IgE antibody (72D) were reacted in 25% BA/PBS for two hours at 37°C. A recombinant antibody obtained by chimerarization of a mouse antihuman IgE antibody (M#9Ch; Japanese Patent Application Laid-open No. 199895/1993) was used as a positive control. The reaction solution was incubated for two hours at room temperature in a 96-well micro-plate in which 100 ng of goat antihuman IgE antibody (Capel Corp.) was immobilized and blocked with 25% BA/PBS. After washing four times with PBST, AP-labeled antihuman IgE (Fc) antibody (Capel Corp.) diluted to 1,000 fold with 25% BA/PBS was added, followed by incubation for two hours at room temperature. After washing four times with PBST, an AP substrate solution was added in the amount of 100 µl per well and the mixture was reacted at room temperature. After terminating the reaction with a 3N-NaOH solution, the absorbance at 405 nm was measured using a micro-plate reader (NJ-2000; Japan Intermed Co., Ltd.). The results are shown in Figure 6. As a result, the antibody 72D was confirmed to possess immune complex forming capability.

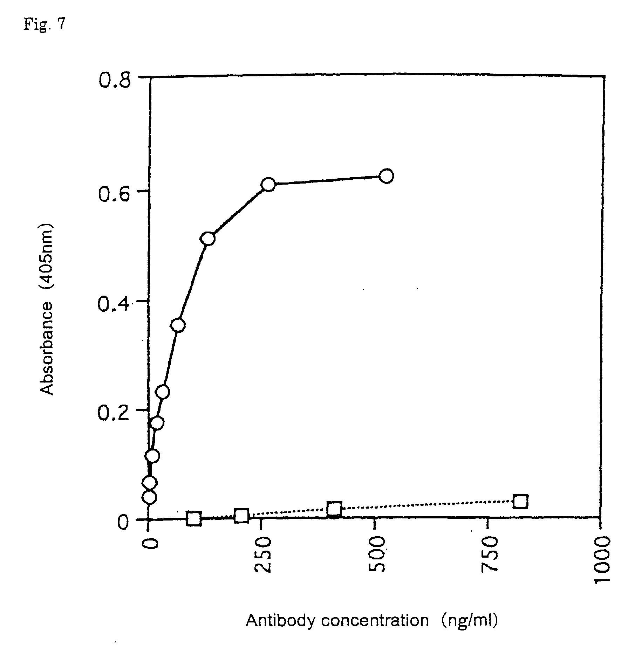

After immobilizing human IgE (100 ng) in a 96-well micro-plate (Nunc Co.; MaxiSorp) by adding the same to each well and allowing to stand overnight at 4°C, then 25% BA/PBS was added to each well. Then, the micro-plate was allowed to stand for two hours at room temperature to complete blocking. After washing four times with PBST, various amounts of the antibody (72D) of the present invention or the above chimerarized recombinant antibody (M#9Ch) were added, followed by incubation for two hours at room temperature. After washing four times with PBST, AP-labeled antihuman IgG (Fc) antibody (Capel Corp.) diluted to 1,000 fold with 25% BA/PBS was added, followed by incubation for two hours at room temperature. After washing four times with PBST, an AP substrate solution was added in the amount of 100 µl per well and the mixture was reacted at room temperature. After terminating the reaction with a 3N-NaOH solution, the absorbance at 405 nm was measured using a micro-plate reader (NJ-2000; Japan Intermed Co., Ltd.). The results are shown in Figure 7. As a result, the antibody of the present invention was confirmed not to bind to IgE directly immobilized onto a micro-plate.

### (2) Binding to IgE-producing cells

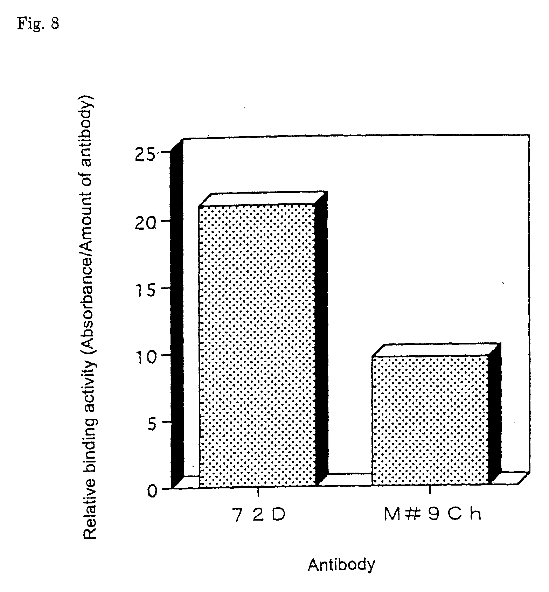

IgE-producing myeloma cell strain U266B1 (ATCC TIB-196) grown in 10% FCS-containing RPMI-1640 culture medium was collected by centrifugation (100xg for 10 minutes) and cells were suspended in an ExCell 301 culture medium to a concentration of 5x10⁶ cells/ml. 100 µl of the cell suspension, 400 µl of antibody (72D or M#9Ch) solution, and 50 µl of 1% BSA/PBS/0.02% NaN₃ was added in a 1.5 ml Eppendorf tube and the mixture was incubated for two hours at room temperature while mixing from time to time. After washing two times by centrifugation with PBS, 100µl of AP-labeled antihuman IgG (Fc) antibody (Capel Corp.) diluted to 1,000 fold with 1% BSA/PBS/0.02% NaN₃ was added, followed by reaction for two hours at room temperature while mixing from time to time. After washing three times by centrifugation with PBS, 200 µl of an AP substrate solution containing sodium p-nitrophenyl phosphate at a concentration of 3 mg/ml was added, followed by reaction for two hours at room temperature while mixing from time to time. 150 µl of the supernatant liquid obtained by centrifugation (10,000 x g for 5 minutes at 4°C) was transferred to a 96 well micro-plate to measure absorbance at 405 nm using a micro-plate reader. The results are shown in Figure 8.

### Example 20

### <Decrease of the free IgE concentration in blood by the antibody of the present invention (experiment for confirming in vivo pharmaceutical effect)>

Purified human IgE (Scripps Inc.) was intravenously administered (50 µg/kg) to male Wistar rats (age: 9 weeks, Japan Charlesriver Co., Ltd.). After five minutes, blood serum before administration of the antihuman IgE antibody was prepared from blood collected from the orbit. 10 minutes after administration of IgE, the antibody (72D) of the present invention dissolved in 0.1% BSA/PBS and the vehicle (0.1% BSA/PBS) were intravenously administered. A recombinant antibody obtained by chimerarization of a mouse antihuman IgE antibody (M#9Ch; Japanese Patent Application Laid-open No. 199895/1993) was used as a positive control. The antibody of the present invention was administered at doses of 100, 300, and 1,000 µg/kg, whereas M#9ch was administered at doses of 10, 100, and 1,000 µg/kg. The blood was collected from the orbit at 15 minutes, 30 minutes, 1 hour, 2 hours, 4 hours, 6 hours, 8 hours, 10 hours, 12 hours, and 24 hours after administration of the IgE. The blood serum prepared by centrifugation was stored at -30°C until used for measurement. Measurement of free IgE concentration was carried out as follows. As immobilizing antibodies, antihuman IgE antibody and polyclonal antihuman IgE antibody (Dako Japan, Co., Ltd.), each dissolved in 50 mM NaHCO₃ (pH 9.6) to a concentration of 2 µg/ml, were added to a 96-well micro-plate in an amount of 100 µl per well. The micro-plate was allowed to stand overnight at 4°C to immobilize the antibodies. The sample was blocked by adding 300 µl of 2% BSA/PBS per well and allowing the micro-plate to stand for one hours at room temperature. Then, 100 µl/well of blood serum sample diluted with 25% BA/PBS was added, followed by incubation for two hours at room temperature. A well to which a standard IgE with a known concentration was added was prepared at the same time for the purpose of preparing a calibration curve. After washing six times with PBST, 100 µl/well of AP labeled goat antihuman IgE antibody (Capel Corp.) diluted to 5,000 fold was added as a secondary antibody, and allowed to stand for two hours at room temperature. After washing six times with PBST, an AP substrate solution was added in the amount of 100 µl per well and the mixture was reacted at room temperature. After terminating the reaction with a 3N-NaOH solution, the absorbance at 405 nm was measured using a micro-plate reader (NJ-2000; Japan Intermed Co., Ltd.) to prepare a calibration curve. The free IgE concentration in the blood serum was quantified using the calibration curve. The results are shown in Table 6 and Figure 9. As a result, a dose-dependent decrease in the blood IgE concentration was induced by the administration of the antibody 72D of the present invention. IgE in the blood has completely disappeared at a dose of 1,000 µg/kg.

**Table 6**

| Antibody (µg/kg) | Blood serum IgE AUC (ng • ml⁻¹ • hr) |
|---|---|
| Vehicle | 5307±574 |
| M#9Ch ( 10) | 2536±577 |
| M#9Ch ( 100) | 2128±193 |
| M#9Ch (1000) | 1009±279 |
| 72D ( 10) | 1695±224 |
| 72D ( 300) | 289±255 |
| 72D (1000) | 0±0 |
| (Data is shown by the mean value ± standard deviation (n=3)) | |

### Example 21

### <Serotonin release test>

40x10⁵ rat basophil leukemia cells (RBL-1; ATCC CRL-1378) were suspended in 4 ml of 0.1% BSA/PBS/0.02% NaN₃. After the addition of human IgE to a concentration of 1 µg/ml, the mixture was incubated for 30 minutes at 4°C. After washing with 0.1% BSA/PBS/0.02% NaN₃, the mixture was suspended in 8 ml of a dilution solution attached to a kit. The suspension was put into Eppendorf tubes in an amount of 0.5 ml per tube. A normal antibody (Capel Corp.) or the antibody of the present invention (72D) was added to each tube to a concentration of 10 µg/ml and the mixture was incubated for 30 minutes at 37°C. The mixture was centrifuged (100G for 5 minutes) to obtain a supernatant as a sample. Released serotonin was quantified using a serotonin measurement kit (Immunotech Co.). The results are shown in Table 7.

The results indicate that no statistical difference (t-test) was seen with regard to release of serotonin by the addition of normal human IgG or the antibody of the present invention as compared with a sample without the addition of antibodies. Therefore, the antibody of the present invention (72D) has been confirmed not to induce degranulation from IgE sensitized basophiles.

**Table 7**

| Antibody | Released serotonin (nM) |
|---|---|
| Vehicle | 91.50±11.02 |
| Normal IgE (10 µg/ml) | 99.49± 9.38 |
| 72D (10 µg/ml) | 104.97±11.22 |
| (Data is shown by the mean value± standard deviation (n=4)) | |

### Example 22

### <Reactivity of the antibody of the present invention to IgE-treated basophiles>

2.52x10⁷ rat basophil leukemia cells (RBL-1; ATCC CRL-1378) were suspended in 12.6 ml of 0.1% BSA/PBS/0.02% NaN₃ to produce a suspension with a concentration of 2x10⁶ cells/ml. The suspension was put into Eppendorf tubes in an amount of 0.5 ml per tube. Human IgE (Scripps Research Institute) was added to the tubes to a final concentration of 1 µg/ml. The mixture was incubated for 30 minutes at 4°C, washed three times with a sheath liquid (IsoFlo; Beckman Coulter, Inc.) , then suspended again in 0.2 ml of sheath liquid. The antibody of the present invention (72D) was added to the tubes to a final concentration of 0, 1, or 10 µg/ml and the mixture was incubated for 30 minutes at 4°C. After washing three times with the sheath liquid, the mixture was suspended in 0.5 ml of FITC-labeled anti-IgG antibody (fluorescein-conjugated goat F (ab') 2 fragment to human IgGFc; Capel Corp.) diluted to 1,000 fold, followed by incubation for 30 minutes at 4°C. The mixture was washed three times with the sheath liquid, suspended again in 1 ml of the same liquid, and subjected to analysis using a flow site meter (EPICSXL; Beckman Coulter, Inc.). The results are shown in Figure 10. As a result, the antibody of the present invention have been confirmed to bind dose-dependently to RBL-1 cells which are pretreated with IgE.

### INDUSTRIAL APPLICABILITY

A novel peptide which binds to a human-type antihuman IgE monoclonal antibody, a method of screening the human-type antihuman IgE monoclonal antibody using the peptide, and the human-type antihuman IgE monoclonal antibody obtained by the method of screening can be provided by the present invention. The novel peptide of the present invention is useful as a tool for screening a human-type antihuman IgE monoclonal antibody possessing high specificity and as a therapeutic agent for allergic diseases. The screening method of the present invention is useful as a method of efficiently obtaining a human-type antihuman IgE monoclonal antibody possessing high specificity. Furthermore, the antibodies obtained by the present invention are useful as a pharmaceutical for preventing and/or treating allergic diseases.

### REMARKS TO DEPOSITED BIOLOGICAL MATERIALS

Name and address of the organization in which the biological materials have been deposited:
Name: The Biotechnology Laboratory, National Institute of Advanced Science and Technology, the Ministry of Economy, Trade and Industry
Address: 1-1-3, Higashi, Tsukuba-shi, Ibaraki-ken, Japan (Postal Code: 305-3566).
Date of deposition: January 27, 1999
Number of deposition given by the deposition organization: FERM BP-7344

## Claims

1. A peptide having an amino acid sequence of Sequence ID No. 1 of the Sequence Table.

2. A method for screening a human-type antihuman IgE monoclonal antibody comprising using the peptide according to claim 1.

3. The method for screening according to claim 2, comprising sensitizing lymphocytes *in vitro* using IgE, establishing a cell strain producing a human-type antihuman IgE antibody by the hybridoma method, examining the affinity of the cell to the peptide having the amino acid sequence of Sequence ID No . 1 in the sequence table, selecting cells producing an antibody with high antigen specificity, and obtaining a human-type antihuman IgE monoclonal antibody.

4. A human-type antihuman IgE monoclonal antibody possessing the following characteristics:
(a) binds to the peptide of claim 1,
(b) specifically binds to human IgE,
(c) selectively binds to human IgE-producing cells, and
(d) exhibits affinity with IgE-sensitized mast cells or basophiles and not induce release of serotonin from basophiles.

5. A DNA encoding the H-chain or L-chain of the human-type antihuman IgE monoclonal antibody of claim 4.

6. A expression product obtained from expression of the DNA of claim 5 using a genetic engineering technique.

7. A human-type antihuman IgE monoclonal antibody described in claim 6 comprising the amino acid sequences of Sequence ID No. 10 (H-chain) and Sequence ID No. 11 (L-chain) of the Sequence Table.

8. A DNA encoding the amino acid sequence described in claim 7.

9. The DNA according to claim 8 comprising the nucleotide sequences of Sequence ID No. 2 (H-chain) and Sequence ID No. 3 (L-chain) of the Sequence Table.

10. A cell in which the DNA of claim 5, 8, or 9 is transfected.

11. A microorganism in which the DNA of claim 5, 8, or 9 is transfected.

12. The microorganism according to claim 11, being the microorganism deposited under number FERM P-17173.

13. A pharmaceutical agent comprising the human-type antihuman IgE monoclonal antibody of claim 4 or 7 as an effective component.

14. A therapeutic agent for allergic diseases comprising the human-type antihuman IgE monoclonal antibody described in claim 4 or 7 as an effective component.
